# EUROPEAN PATENT SPECIFICATION

(11) **EP 1 192 156 B1**
(45) Date of publication and mention of the grant of the patent: **18.02.2004**
(21) Application number: 00946588.1
(22) Date of filing: 15.06.2000
(51) Int. Cl.: C07D 471/08, C07D 471/20, A61K 31/435

(54) **BISPIDINE COMPOUNDS USEFUL IN THE TREATMENT OF CARDIAC ARRHYTHMIAS**
BISPIDIN-DERIVATE ZUR BEHANDLUNG VON HERZARRHYTHMIEN
COMPOSES DE BISPIDINE POUVANT ETRE UTILISES POUR LE TRAITEMENT DE L'ARYTHMIE CARDIAQUE

(30) Priority: 16.06.1999 SE 9902269
(43) Date of publication of application: 03.04.2002
(73) Proprietor: AstraZeneca AB, 151 85 Södertälje (SE)
(72) Inventor: BJÖRSNE, Magnus, S-431 83 Mölndal (SE); FRANTSI, Marianne, S-431 83 Mölndal (SE); HOFFMANN, Kurt-Jürgen, S-431 83 Mölndal (SE); OHLSSON, Bengt, S-431 83 Mölndal (SE)
(86) International application number: PCT/SE2000/001252
(87) International publication number: WO 2000/076998

(56) References cited:
- EP-A2- 0 306 871
- EP-A2- 0 308 843
- WO-A1-91/07405
- WO-A1-99/31100
- US-A- 5 786 481

## Description

### Field of the Invention

This invention relates to novel pharmaceutically useful compounds, in particular compounds which are useful in the treatment of cardiac arrhythmias.

### Background and Prior Art

Cardiac arrhythmias may be defined as abnormalities in the rate, regularity, or site of origin of the cardiac impulse or as disturbances in conduction which causes an abnormal sequence of activation. Arrhythmias may be classified clinically by means of the presumed site of origin (i.e. as supraventricular, including atrial and atrioventricular, arrhythmias and ventricular arrhythmias) and/or by means of rate (i.e. bradyarrhythmias (slow) and tachyarrhythmias (fast)).

In the treatment of cardiac arrhythmias, the negative outcome in clinical trials (see, for example, the outcome of the Cardiac Arrhythmia Suppression Trial (CAST) reported in New England Journal of Medicine, 321, 406 (1989)) with "traditional" antiarrhythmic drugs, which act primarily by slowing the conduction velocity (class I antiarrhythmic drugs), has prompted drug development towards compounds which selectively delay cardiac repolarization, thus prolonging the QT interval. Class III antiarrhythmic drugs may be defined as drugs which prolong the trans-membrane action potential duration (which can be caused by a block of outward K⁺ currents or from an increase of inward ion currents) and refractoriness, without affecting cardiac conduction.

One of the key disadvantages of hitherto known drugs which act by delaying repolarization (class III or otherwise) is that they all are known to exhibit a unique form of proarrhythmia known as *torsades de pointes* (turning of points), which may, on occasion be fatal. From the point of view of safety, the minimisation of this phenomenon (which has also been shown to be exhibited as a result of administration of non-cardiac drugs such as phenothiazines, tricyclic antidepressants, antihistamines and antibiotics) is a key problem to be solved in the provision of effective antiarrhythmic drugs.

Antiarrhythmic drugs based on bispidines (3,7-diazabicyclo[3.3.1]nonanes), are known from *inter alia* international patent application WO 91/07405, European patent applications 306 871, 308 843 and 665 228 and US patents 3,962,449, 4,556,662, 4,550,112, 4,459,301 and 5,468,858, as well as journal articles including *inter alia* J. Med. Chem. **39**, 2559, (1996), Pharmacol. Res., **24**, 149 (1991), Circulation, **90**, 2032 (1994) and Anal. Sci. **9**, 429, (1993). Known bispidine-based antiarrhythmic compounds include bisaramil (3-methyl-7-ethyl-9a,4'-(Cl-benzoyloxy)-3,7-diazabicyclo[3.3.1]nonane), tedisamil (3',7'-bis(cyclopropylmethyl)spiro(cyclopentane-1,9')-3,7-diazabicyclo[3.3.1]nonane), SAZ-VII-22 (3-(4-chlorobenzoyl)-7-isopropyl-3,7-diazabicyclo[3.3.1]nonane), SAZ-VII-23 (3-benzoyl-7-isopropyl-3,7-diazabicyclo[3.3.1]nonane), GLG-V-13 (3-[4-(1H-imidazol-1-yl)benzoyl]-7-isopropyl-3,7-diazabicyclo[3.3.1]nonane), KMC-IV-84 (7-[4'-(1*H*-imidazolo-1-yl)benzenesulfonyl]-3-isopropyl-3,7-diazabicyclo[3.3.1]nonane dihydroperchlorate and ambasilide (3-(4-aminobenzoyl)-7-benzyl-3,7-diazabicyclo[3.3.1]nonane).

We have surprisingly found that a novel group of bispidine-based compounds exhibit electrophysiological activity, preferably class III electrophysiological activity, and are therefore expected to be useful in the treatment of cardiac arrhythmias.

### Disclosure of the Invention

According to the invention there is provided compounds of formula I, 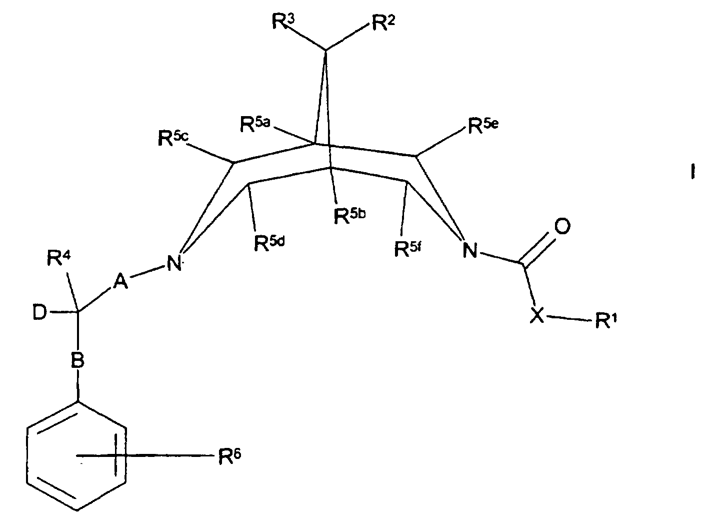 wherein
R¹ represents C₁₋₁₂ alkyl, -(CH₂)ₐ-aryl, or -(CH₂)ₐ-Het¹ (all of which are optionally substituted and/or terminated (as appropriate) by one or more substituents selected from -OH, halo, cyano, nitro, C₁₋₄ alkyl and/or C₁₋₄ alkoxy);
a represents 0, 1, 2, 3, or 4;
Het¹ represents a five to ten-membered heterocyclic ring containing one or more heteroatoms selected from oxygen, nitrogen and/or sulfur, and which also optionally includes one or more = O substituents; X represents O or S;
R^{5a}, R^{5b}, R^{5c}, R^{5d}, R^{5e} and R^{5f} independently represent H or C₁₋₃ alkyl;
R² and R³ independently represent H, C₁₋₄ alkyl (optionally substituted and/or terminated with one or more nitro or cyano groups), OR⁷, N(R^{7a})R^{7b}, OC(O)R⁸ or together form -O-(CH₂)₂-O-, -(CH₂)₃-, -(CH₂)₄- or - (CH₂)₅-;
R⁷ and R⁸ independently represent H, C₁₋₆ alkyl or -(CH₂)_{b}-aryl (which latter two groups are optionally substituted and/or terminated by one or more substituents selected from -OH, halo, cyano, nitro, C₁₋₄ alkyl and/or C₁₋₄ alkoxy);
R^{7a} and R^{7b} independently represent H or C₁₋₆ alkyl;
b represents 0, 1, 2, 3 or 4;
R⁴ represents H or C₁₋₆ alkyl;
D represents H, C₁₋₄ alkyl, -OR⁹, or -(CH₂)_{c}N(R¹⁰)(R¹¹);
R⁹ represents H, C₁₋₆ alkyl, -C(O)R¹², -(CH₂)_{d}-aryl or -(CH₂)_{d}-Het² (which latter three groups are optionally substituted by one or more substituents selected from -OH, halo, cyano, nitro, C₁₋₄ alkyl, C₁₋₄ alkoxy, C(O)R¹³, C(O)OR¹⁴ and/or -N(H)S(O)ₑR¹⁵);
R¹⁰ represents H, C₁₋₆ alkyl, -(CH₂)_{f}-aryl, -C(NH)NH₂, -S(O)₂R^{15a}, -[C(O)]_{g}N(R¹⁶)(R¹⁷), -C(O)R¹⁸ or -C(O)OR¹⁹;
e represents 0, 1 or 2;
g represent 1 or 2;
R¹¹ represents H, C₁₋₆ alkyl, -C(O)R²⁰ or -(CH₂)ₕ-aryl (which latter group is optionally substituted and/or terminated (as appropriate) by one or more substituents selected from -OH, cyano, halo, amino, nitro, C₁₋₆ alkyl and/or C₁₋₆ alkoxy);
R¹², R¹³, R¹⁴, R¹⁶, R¹⁷, R¹⁸, R¹⁹ and R²⁰ independently represent H, C₁₋₆ alkyl, Het³ or -(CH₂)ⱼ-aryl (which latter three groups are optionally substituted and/or terminated (as appropriate) by one or more substituents selected from -OH, cyano, halo, amino, nitro, C₁₋₆ alkyl and/or C₁₋₆ alkoxy);
R¹⁵ and R^{15a} independently represent C₁₋₆ alkyl, aryl or -(CH₂)ₖ-aryl (all of which are all optionally substituted and/or terminated (as appropriate) by one or more substituents chosen from halo, nitro, C₁₋₆ alkyl and/or C₁₋₆ alkoxy);
c, d, f, h, j and k independently represent 0, 1, 2, 3 or 4;
Het² and Het³ independently represent five to ten-membered heterocyclic rings containing one or more heteroatoms selected from oxygen, nitrogen and/or sulfur, and which also optionally includes one or more =O substituents;
R⁶ represents one or more optional substituents selected from -OH, cyano, halo, amino, nitro, C₁₋₆ alkyl (optionally terminated by N(H)C(O)OR^{20a}), C₁₋₆ alkoxy, -C(O)N(H)R²¹, -NHC(O)N(H)R²², -N(H)S(O)₂R²³ and/or -OS(O)₂R²⁴;
R²¹ and R²² independently represent H or C₁₋₆ alkyl;
R^{20a}, R²³ and R²⁴ independently represent C₁₋₆ alkyl;
A represents a single bond, C₁₋₆ alkylene, -N(R²⁵)(CH₂)ₘ-, -O(CH₂)ₘ- or -(CH₂)ₘC(H)(OR²⁵)(CH₂)ₙ- (in which latter three groups, the -(CH₂)ₘ-group is attached to the bispidine nitrogen atom and which latter four groups are optionally substituted by one or more -OH groups);
B represents a single bond, C₁₋₄ alkylene, -(CH₂)ₚN(R²⁶)-, -(CH₂)ₚS(O)_{q}-, - (CH₂)ₚO- (in which three latter groups, the -(CH₂)ₚ- group is attached to the carbon atom bearing D and R⁴), -C(O)N(R²⁶)- (in which latter group, the -C(O)- group is attached to the carbon atom bearing D and R⁴), -N(R²⁶)C(O)O(CH₂)ₚ- or -N(R²⁶)(CH₂)ₚ- (in which latter two groups, the N(R²⁶) group is attached to the carbon atom bearing D and R⁴);
m, n and p independently represent 0, 1, 2, 3 or 4;
q represents 0, 1 or 2;
R²⁵ represents H, C₁₋₆ alkyl or C(O)R²⁷;
R²⁶ represents H or C₁₋₆ alkyl;
R²⁷ represents H, C₁₋₆ alkyl, Het⁴ or -(CH₂)ᵣ-aryl (which latter two groups are optionally substituted and/or terminated (as appropriate) by one or more substituents selected from -OH, cyano, halo, amino, nitro, C₁₋₆ alkyl and/or C₁₋₆ alkoxy);
Het⁴ represents a five to ten-membered heterocyclic ring containing one or more heteroatoms selected from oxygen, nitrogen and/or sulfur, and which also optionally includes one or more =O substituents;
r represents 0, 1, 2, 3 or 4;
or a pharmaceutically acceptable derivative thereof;
provided that:
(a) R^{5a}, R^{5b}, R^{5c}, R^{5d}, R^{5e} and R^{5f} do not all simultaneously represent H;
(b) R^{5a} and R^{5b} do not represent C₁₋₃ alkyl when R^{5c}, R^{5d}, R^{5e} and R^{5f} all represent H; and
(c) when D represents -OH or -(CH₂)_{c}N(R¹⁰)R¹¹ in which c represents 0, then:-
   (i) A does not represent -N(R²⁵)(CH₂)ₘ-, -O(CH₂)ₘ- or -(CH₂)ₘC(H)(OR²⁵)(CH₂)ₙ- (in which n is 0); and/or
   (ii) p does not represent 0 when B represents -(CH₂)ₚN(R²⁶)-, - (CH₂)ₚS(O)_{q}- or -(CH₂)ₚO-,
which compounds are referred to hereinafter as "the compounds of the invention".

Aryl groups that may be mentioned include C₆₋₁₀ aryl groups, such as phenyl, naphthyl and the like. Oxyaryl groups that may be mentioned include C₆₋₁₀ oxyaryl groups, such as oxyphenyl (phenoxy), oxynaphthyl (naphthoxy) and the like. When substituted, aryl and aryloxy groups are preferably substituted by between one and three substituents.

Het¹, Het², Het³ and Het⁴ groups that may be mentioned include those containing 1 to 4 heteroatoms (selected from the group oxygen, nitrogen and/or sulfur) and in which the total number of atoms in the ring system is between five and ten. Het (Het¹, Het², Het³ and Het⁴) groups may be wholly/partly aromatic in character and may be bicyclic. Heterocyclic groups that may be mentioned include morpholinyl, thiazolyl, oxazolyl, isoxazolyl, cinnolinyl, quinazolinyl, phthalazinyl, purinyl, benzimidazolyl, pyrimindinyl, piperazinyl, pyrazinyl, piperidinyl, pyridinyl, pyrrolinyl, pyrrolidinyl, pyrollidinonyl, triazolyl, imidazolyl, quinolinyl, isoquinolinyl, dioxanyl, benzodioxanyl, benzodioxolyl, benzodioxepanyl, benzomorpholinyl, indolyl, pyrazolyl, pyrrolyl, benzothiophenyl, thiophenyl, chromanyl, thiochromanyl, benzofuranyl, pyranyl, tetrahydropyranyl, tetrahydrofuranyl, furanyl and the like. Substituents on Het (Het¹, Het², Het³ and Het⁴) groups may, where appropriate, be located on any atom in the ring system including a heteroatom. The point of attachment of Het (Het¹, Het², Het³ and Het⁴) groups may be *via* any atom in the ring system including (where appropriate) a heteroatom. Het (Het¹, Het², Het³ and Het⁴) groups may optionally be in the N- or S-oxidised form.

Pharmaceutically acceptable derivatives include salts and solvates. Salts which may be mentioned include acid addition salts. Pharmaceutically acceptable derivatives also include C₁₋₄ alkyl quaternary ammonium salts and N-oxides, provided that, when a N-oxide is present:
(a) no Het (Het¹, Het², Het³, Het⁴) groups contain an unoxidised S-atom;
(b) X does not represent S;
(c) q does not represent 0, when B represents -(CH₂)ₚS(O)_{q}-; and/or
(d) e does not represent 0, when R⁹ is substituted by N(H)S(O)ₑR¹⁵.

The compounds of the invention may exhibit tautomerism. All tautomeric forms and mixtures thereof are included within the scope of the invention.

The compounds of the invention may also contain one or more asymmetric carbon atoms and may therefore exhibit optical and/or diastereoisomerism. Diastereoisomers may be separated using conventional techniques, e.g. chromatography or fractional crystallisation. The various stereoisomers may be isolated by separation of a racemic or other mixture of the compounds using conventional, e.g. fractional crystallisation or HPLC, techniques. Alternatively the desired optical isomers may be made by reaction of the appropriate optically active starting materials under conditions which will not cause racemisation or epimerisation, or by derivatisation, for example with a homochiral acid followed by separation of the diastereomeric esters by conventional means (e.g. HPLC, chromatography over silica). All stereoisomers are included within the scope of the invention.

Alkyl groups that R¹, R², R³, R⁴, R^{5a}, R^{5b}, R^{5c}, R^{5d}, R^{5e}, R^{5f}, R⁶, R⁷, R^{7a}, R^{7b}, R⁸, R⁹, R¹⁰, R¹¹, R¹², R¹³, R¹⁴, R¹⁵, R^{15a}, R¹⁶, R¹⁷, R¹⁸, R¹⁹, R²⁰, R^{20a}, R²¹, R²², R²³, R²⁴, R²⁵, R²⁶, R²⁷ and D may represent, and with which R¹, R⁷, R⁸, R⁹, R¹¹, R¹², R¹³, R¹⁴, R¹⁵, R^{15a}, R¹⁶, R¹⁷, R¹⁸, R¹⁹, R²⁰ and R²⁷ may be substituted; and alkoxy groups that R⁶ may represent, and with which R¹, R⁷, R⁸, R⁹, R¹¹, R¹², R¹³, R¹⁴, R¹⁵, R^{15a}, R¹⁶, R¹⁷, R¹⁸, R¹⁹, R²⁰ and R²⁷ may be substituted, may be linear or, when there is a sufficient number (i.e. three) of carbon atoms, be branched and/or cyclic. Further, when there is a sufficient number (i.e. four) of carbon atoms, such alkyl and alkoxy groups may also be part cyclic/acyclic. Such alkyl and alkoxy groups may also be saturated or, when there is a sufficient number (i.e. two) of carbon atoms, be unsaturated and/or interrupted by oxygen and/or substituted by one or more fluoro groups.

Alkylene groups that A and B may represent, and -(CH₂)- containing groups that R¹, R² and R³ (together), R⁷, R⁸, R⁹, R¹⁰, R¹¹, R¹², R¹³, R¹⁴, R¹⁵, R^{15a}, R¹⁶, R¹⁷, R¹⁸, R¹⁹, R²⁰, R²⁷, A, B and D may include, may be linear or, when there is a sufficient number (i.e. two) of carbon atoms, be branched. Such alkylene groups and -(CH₂)- containing chains may also be saturated or, when there is a sufficient number (i.e. two) of carbon atoms, be unsaturated and/or interrupted by oxygen.

As used herein, the term "halo" includes fluoro, chloro, bromo or iodo.

Abbreviations are listed at the end of this specification.

According to a further aspect of the invention there is provided compounds of formula I as hereinbefore defined, but with the further provisos that:
(a) when A represents -N(R²⁵)(CH₂)ₘ- or -O(CH₂)ₘ-, then m does not represent 0 or 1; and
(b) when D represents -OH or -(CH₂)_{c}N(R¹⁰)R¹¹ in which c represents 0, then B does not represent -N(R²⁶)C(O)O(CH₂)ₚ- or -N(R²⁶)(CH₂)ₚ-.

### Preferred compounds of the invention include those in which:

R¹ represents optionally substituted -(CH₂)ₐ-phenyl in which a is 0, 1, 2 or 3, or, preferably, optionally substituted, optionally unsaturated, linear, branched or cyclic, C₁₋₈ alkyl (which latter group may also be interrupted by an oxygen atom);
R² represents H;
R³ represents H;
R⁴ represents H or C₁₋₃ alkyl;
R^{5a} and R^{5b} either both represent H or both represent methyl;
R^{5c}, R^{5d}, R^{5e} and R^{5f} independently represent H or C₁₋₂ alkyl;
R⁶ represents one or more substituents selected from C₁₋₆ alkyl (which alkyl group is optionally terminated by a N(H)C(O)OR^{20a} group (in which R^{20a} represents C₁₋₅ alkyl)), cyano, nitro, amino, C(O)N(H)R²¹ and/or
-N(H)S(O)₂R²³;
X represents O;
A represents a single bond or linear, or branched, C₁₋₄ alkylene (which group is also optionally interrupted by O);
B represents a single bond, C₁₋₄ alkylene, -(CH₂)ₚO- or -(CH₂)ₚN(R²⁶)- (in which latter two cases p is 1, 2 or 3);
D represents H, OR⁹ (in which R⁹ represents H, C₁₋₃ alkyl or optionally substituted phenyl) or N(H)R¹⁰ (in which R¹⁰ represents H or C₁₋₄ alkyl);
when the bispidine nitrogen bearing A optionally bears a C₁₋₄ alkyl group, thus forming a quaternary ammonium salt, the alkyl group is a methyl group.

More preferred compounds of the invention include those in which:
R¹ represents linear or branched C₂₋₆ alkyl;
R⁴ represents H;
R^{5a} and R^{5b} both represent H;
R⁶ represents cyano, preferably in the *para* position relative to B;
A represents C₁₋₄ alkylene;
B represents a single bond or -(CH₂)ₚO- (in which p is 1 or 2);
D represents H, OH, NH₂ or phenoxy (optionally substituted on the phenyl ring by one or more C₁₋₃ alkoxy groups).

Preferred compounds of the invention include the compounds of Examples described hereinafter.

### Preparation

According to the invention there is also provided a process for the preparation of compounds of formula I which comprises:
(a) reaction of a compound of formula II, 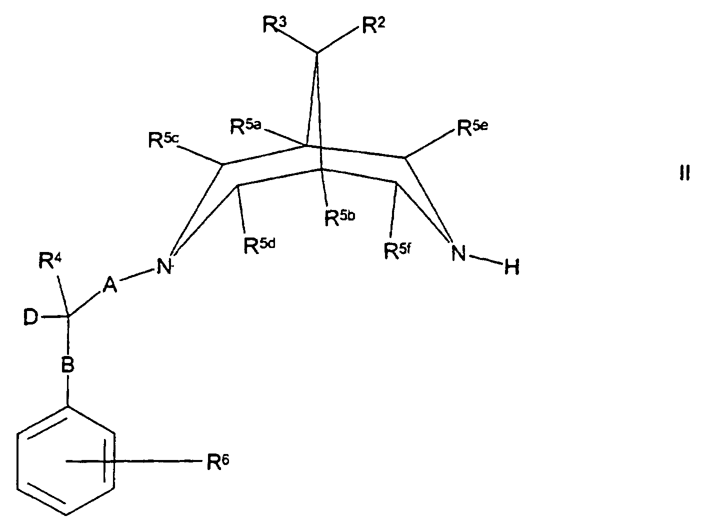 wherein R², R³, R⁴, R^{5a}, R^{5b}, R^{5c}, R^{5d}, R^{5e}, R^{5f}, R⁶, A, B and D are as hereinbefore defined with a compound of formula III,

   R¹XC(O)L¹ III

   wherein L¹ represents a leaving group, such as Hal, imidazolyl or -OC(O)XR¹, Hal represents Cl, Br or I, and R¹ and X are as hereinbefore defined, for example at or above room temperature in the presence of a suitable base (e.g. aqueous NaOH, K₂CO₃ or triethylamine) and an appropriate organic solvent (e.g. CH₂Cl₂, THF, acetonitrile, toluene, or mixtures of such solvents);
(b) for compounds of formula I in which A represents CH₂ and D represents - OH or -N(H)R¹⁰, wherein R¹⁰ is as hereinbefore defined, reaction of a compound of formula IV, 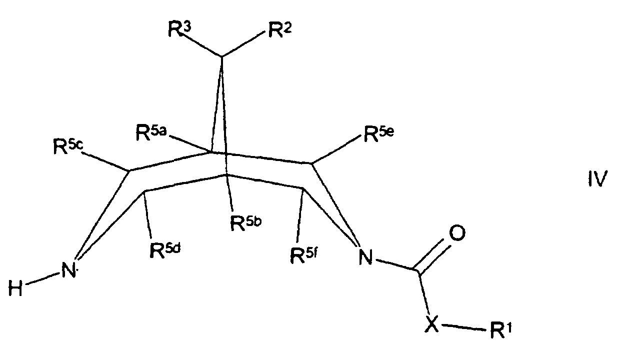 wherein R¹, R², R³, R^{5a}, R^{5b}, R^{5c}, R^{5d}, R^{5e}, R^{5f} and X are as hereinbefore defined, with a compound of formula V, 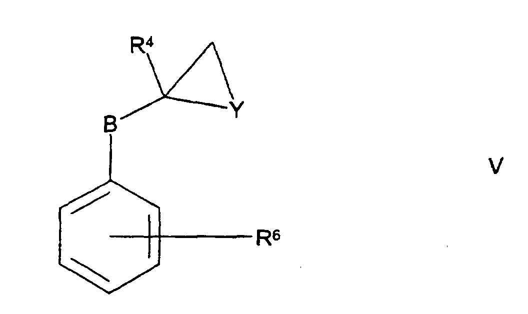 wherein Y represents O or N(R¹⁰) and R⁴, R⁶, R¹⁰ and B are as hereinbefore defined, for example at elevated temperature (e.g. 60°C to reflux) in the presence of a suitable solvent (e.g. a lower alkyl alcohol (e.g. IPA), acetonitrile, or a mixture of a lower alkyl alcohol and water);
(c) reaction of a compound of formula IV, as hereinbefore defined, with a compound of formula VI, 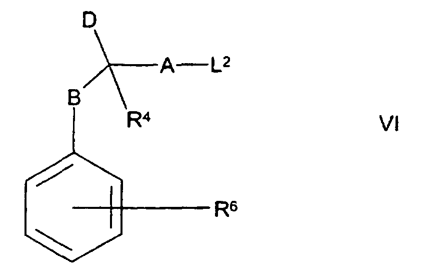 wherein L² represents a leaving group (e.g. mesylate, tosylate or Hal, where Hal is as hereinbefore defined) and R⁴, R⁶, A, B and D are as hereinbefore defined, for example at elevated temperature (e.g. between 35°C and reflux temperature) in the presence of a suitable base (e.g. triethylamine or K₂CO₃) and an appropriate organic solvent (e.g. acetonitrile or dimethylsulfoxide);
(d) for compounds of formula I in which D represents H or OH and R⁴ represents H, reduction of a compound of formula VII, 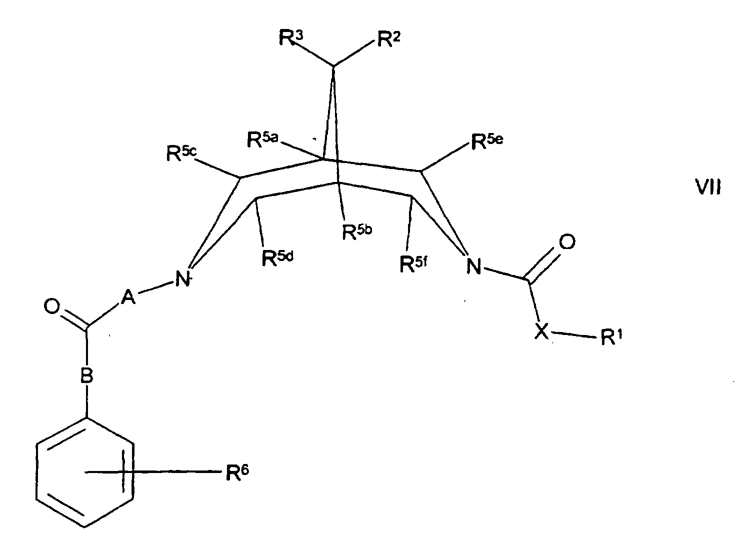 wherein R¹, R², R³, R^{5a}, R^{5b}, R^{5c}, R^{5d}, R^{5e}, R^{5f}, R⁶, A, B and X are as hereinbefore defined, in the presence of a suitable reducing agent and under appropriate reaction conditions; for example, for formation of compounds of formula I in which D represents -OH, reduction may be performed under mild reaction conditions in the presence of e.g. sodium borohydride and an appropriate organic solvent (e.g. THF); and for formation of compounds of formula I in which D represents H, reduction may be performed by activating the relevant C=O group using an appropriate agent (such as tosylhydrazine) in the presence of a suitable reducing agent (e.g. sodium borohydride or sodium cyanoborohydride) and an appropriate organic solvent (e.g. a lower alkyl alcohol);
(e) for compounds of formula I in which R² and R³ both represent H, reduction of a corresponding compound of formula VIII, 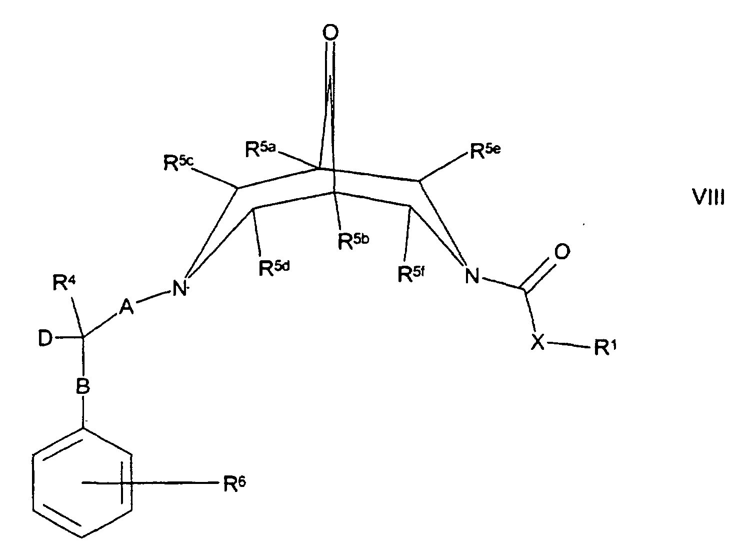 wherein R¹, R⁴, R^{5a}, R^{5b}, R^{5c}, R^{5d}, R^{5e}, R^{5f}, R⁶, A, B, D and X are as hereinbefore defined, and in which the bridgehead C=O group may be activated using an appropriate agent, such as tosylhydrazine, in the presence of a suitable reducing agent (e.g. sodium borohydride or sodium cyanoborohydride) and an appropriate organic solvent (e.g. a lower alkyl alcohol); when the C=O group is activated, the activation step may be carried out at between room and reflux temperature in the presence of an appropriate organic solvent (e.g. a lower alkyl alcohol such as methanol, ethanol or IPA), whereafter the reducing agent may be added to the reaction mixture and the reduction carried out at between 60°C and reflux, advantageously in the presence of a suitable organic acid (e.g. acetic acid);
(f) for compounds of formula I in which one of R² and R³ represents H and the other represents -OH, reduction of a corresponding compound of formula VIII, as hereinbefore defined, in the presence of a mild reducing agent, e.g. sodium borohydride, and an appropriate organic solvent (e.g. a lower alcohol such as methanol or ethanol);
(g) for compounds of formula I in which R² and/or R³ represent OC(O)R⁸ and R⁸ is as hereinbefore defined, coupling of a corresponding compound of formula I in which R² and/or R³ (as appropriate) represent OH and a compound of formula VIIIA,

   R⁸CO₂H VIIIA

   wherein R⁸ is as hereinbefore defined, for example at ambient temperature (e.g. 25°C) in the presence of a suitable coupling agent (e.g. 1-(3-dimethylaminopropyl)-3-ethylcarbodiimide), an appropriate catalyst (e.g. 4-dimethylaminopyridine) and a reaction-inert organic solvent (e.g. THF);
(h) for compounds of formula I in which D represents -(CH₂)_{c}NH₂, reduction of a corresponding compound of formula IX, 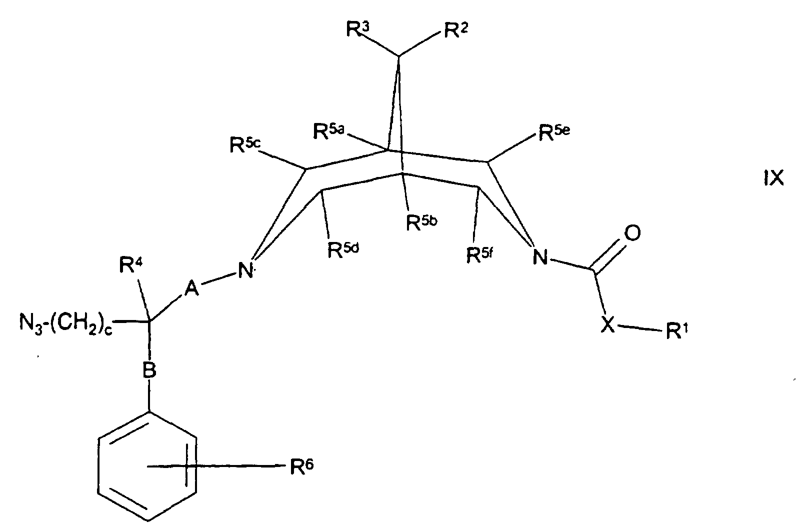 wherein c, R¹, R², R³, R⁴, R^{5a}, R^{5b}, R^{5c}, R^{5d}, R^{5e}, R^{5f}, R⁶, A, B and X are as hereinbefore defined, for example by hydrogenation at a suitable pressure in the presence of a suitable catalyst (e.g. palladium on carbon) and an appropriate solvent (e.g. a water-ethanol mixture);
(i) for compounds of formula I in which D represents -N(R¹¹)C(O)NH(R¹⁷), in which R¹¹ and R¹⁷ are as hereinbefore defined, except that R¹¹ does not represent C(O)R²⁰, reaction of a corresponding compound of formula I in which D represents -N(R¹¹)H, in which R¹¹ is as hereinbefore defined except that is does not represent C(O)R²⁰ in which R²⁰ is as hereinbefore defined, with a compound of formula X,

   R¹⁷N=C=O X

   wherein R¹⁷ is as hereinbefore defined, for example at ambient temperature (e.g. 25°C) in the presence of a suitable solvent (e.g. benzene);
(j) for compounds of formula I in which D represents -N(H)[C(O)]₂NH₂, reaction of a corresponding compound of formula I in which D represents -NH₂ with oxalic acid diamide, for example at between -10 and 25°C in the presence of a suitable coupling agent (e.g. 1-(3-dimethylaminopropyl)-3-ethylcarbodiimide), an appropriate activating agent (e.g. 1-hydroxybenzotriazole), a suitable base (e.g. triethylamine) and a reaction-inert organic solvent (e.g. DMF);
(k) for compounds of formula I in which D represents -N(R¹¹)C(O)R¹⁸, in which R¹¹ and R¹⁸ are as hereinbefore defined, except that R¹¹ does not represent C(O)R²⁰, reaction of a corresponding compound of formula I in which D represents -N(R¹¹)H, in which R¹¹ is as hereinbefore defined except that it does not represent C(O)R²⁰, with a compound of formula XI,

   R¹⁸C(O)R^{x} XI

   wherein R^{λ} represents a suitable leaving group, such as C₁₋₄ alkoxy, Hal (e.g. Cl, Br) or *p*-nitrophenyl and R¹⁸ is as hereinbefore defined, for example at between ambient and reflux temperature in the presence of a suitable solvent (e.g. methanol or DMSO) and (as appropriate) a suitable base (e.g. K₂CO₃ or TEA);
(l) for compounds of formula I in which D represents -N(H)R¹⁰ and R¹⁰ is as hereinbefore defined except that it does not represent H or -C(NH)NH₂, reaction of a corresponding compound of formula I wherein D represents -NH₂ with a compound of formula XIA,

   R^{10a}L¹ XIA

   wherein R^{10a} represents R¹⁰ as hereinbefore defined, except that it does not represent H or -C(NH)NH₂ and L¹ is as hereinbefore defined, for example under conditions that are known to those skilled in the art;
(m) for compounds of formula I which are bispidine-nitrogen N-oxide derivatives, oxidation of the corresponding bispidine nitrogen of a corresponding compound of formula I, in the presence of a suitable oxidising agent (e.g. *m*-chloroperbenzoic acid), for example at 0°C in the presence of a suitable organic solvent (e.g. DCM);
(n) for compounds of formula I which are C₁₋₄ alkyl quaternary ammonium salt derivatives, in which the alkyl group is attached to a bispidine nitrogen, reaction, at the bispidine nitrogen, of a corresponding compound of formula I with a compound of formula XII,

   R^{a}Hal XII

   wherein R^{a} represents C₁₋₄ alkyl and Hal is as hereinbefore defined, for example at room temperature in the presence of an appropriate organic solvent (e.g. DMF), followed by purification (using e.g. HPLC) in the presence of a suitable counter-ion provider (e.g. NH₄OAc);
(o) for compounds of formula I in which D and R⁴ both represent H, A represents C₁₋₆ alkylene, B represents -N(R¹⁶)(CH₂)ₚ- and R²⁶ and p are as hereinbefore defined, reaction of a compound of formula XIII, 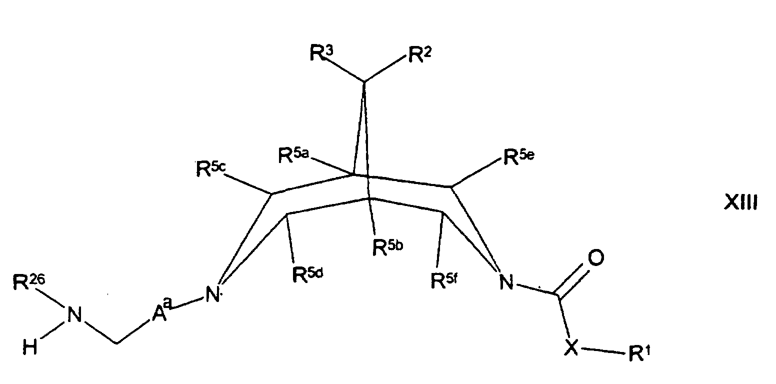 wherein A^{a} represents C₁₋₆ alkylene and R¹, R², R³, R^{5a}, R^{5b}, R^{5c}, R^{5d}, R^{5e}, R^{5f}, R²⁶ and X are as hereinbefore defined with a compound of formula XIV , 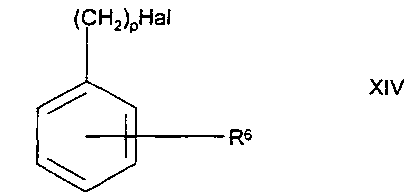 wherein R⁶, p and Hal are as hereinbefore defined, for example at 40°C in the presence of a suitable organic solvent (e.g. acetonitrile);
(p) reaction of a compound of formula II, as hereinbefore defined, with a compound of formula XV,

   R¹XH XV

   wherein R¹ and X are as hereinbefore defined, in the presence of 1,1'-carbonyldiimidazole, for example by refluxing in the presence of a suitable organic solvent (e.g. THF);
(q) for compounds of formula I in which R⁹ represents optionally substituted C₁₋₆ alkyl, optionally substituted -(CH₂)_{d}-aryl or optionally substituted - (CH₂)_{d}-Het²-, reaction of a corresponding compound of formula I, in which D represents OH with a compound of formula XVI,

   R^{9a}OH XVI

   wherein R^{9a} represents optionally substituted C₁₋₆ alkyl, optionally substituted -(CH₂)_{d}-aryl or optionally substituted -(CH₂)_{d}-Het² and d and Het² are as hereinbefore defined, for example at between ambient (e.g. 25°C) and reflux temperature, under Mitsunobu-type conditions (i.e. in the presence of e.g. triphenylphosphine, an azodicarboxylate derivative (e.g. 1,1'-(azodicarbonyl)dipiperidine) and a suitable organic solvent (e.g. dichloromethane));
(r) for compounds of formula I in which R⁹ represents optionally substituted C₁₋₆ alkyl, optionally substituted -(CH₂)_{d}-aryl or optionally substituted -(CH₂)_{d}-Het², reaction of a compound of formula XVII, 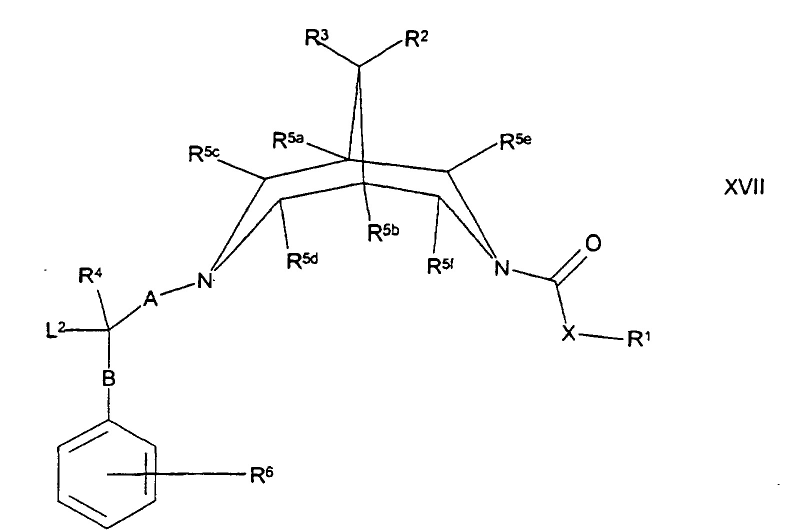 wherein L², R¹, R², R³, R⁴, R^{5a}, R^{5b}, R^{5c}, R^{5d}, R^{5e}, R^{5f}, R⁶, X, A and B are as hereinbefore defined with a compound of formula XVI as hereinbefore defined, for example at between ambient (e.g. 25°C) and reflux temperature, under Williamson-type conditions (i.e. in the presence of an appropriate base (e.g. KOH or NaH) and a suitable organic solvent (e.g. dimethylsulfoxide or DMF));
(s) for compounds of formula I in which R⁹ represents C(O)R¹² and R¹² is as hereinbefore defined, reaction of a corresponding compound of formula I in which D represents OH with a compound of formula XVIII,

   R¹²CO₂H XVIII

   wherein R¹² is as hereinbefore defined, for example at ambient temperature (e.g. 25°C) in the presence of a suitable coupling agent (e.g. 1-(3-dimethylaminopropyl)-3-ethylcarbodiimide), an appropriate catalyst (e.g. 4-dimethylaminopyridine) and a reaction-inert organic solvent (e.g. THF);
(t) for compounds of formula I in which one or both of R² and R³ represent -N(R^{7a})R^{7b} in which one or both of R^{7a} and R^{7b} represents C₁₋₆ alkyl, alkylation of a corresponding compound of formula I in which R² and/or R³ represent -N(R^{7a})R^{7b} (as appropriate) in which R^{7a} and/or R^{7b} (as appropriate) represent H, using a compound of formula XVIIIA,

   R^{7c}L¹ XVIIIA

   wherein R^{7c} represents C₁₋₆ alkyl and L¹ is as hereinbefore defined, for example under conditions that are well known to those skilled in the art; or
(u) conversion of one R⁶ substituent to another using techniques well known to those skilled in the art.

Compounds of formula II may be prepared by reaction of a compound of formula XIX, 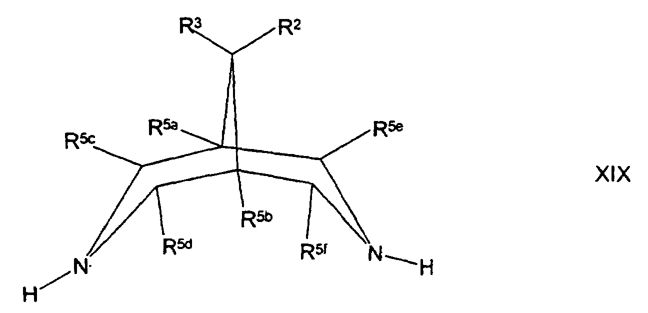 wherein R², R³, R^{5a}, R^{5b}, R^{5c}, R^{5d}, R^{5e} and R^{5f} are as hereinbefore defined, with a compound of formula VI as hereinbefore defined, for example as described hereinbefore for synthesis of compounds of formula I (process step (c)).

Compounds of formula II in which A represents CH₂ and D represents OH or N(H)R¹⁰ may be prepared by reaction of a compound of formula XIX, as hereinbefore defined with a compound of formula V as hereinbefore defined, for example as described hereinbefore for synthesis of compounds of formula I (process step (b)).

Compounds of formula II in which R² and R³ both represent H may be prepared by reduction of a compound of formula XX, 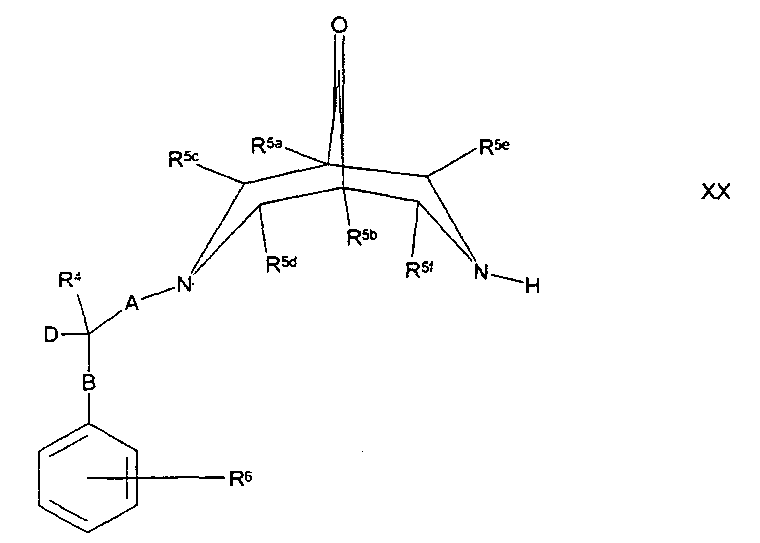 wherein R⁴, R^{5a}, R^{5b}, R^{5c}, R^{5d}, R^{5e}, R^{5f}, R⁶, A, B and D are as hereinbefore defined, and in which the C=O group may be activated using an appropriate agent, such as tosylhydrazine, for example as described hereinbefore for synthesis of compounds of formula I (process step (e)).

Compounds of formula II in which R² represents OH and R³ represents optionally substituted C₁₋₄ alkyl, may be prepared by reaction of a compound of formula XX, or a protected derivative thereof, with a compound of formula XXI,

R^{3a}MgHal XXI

wherein R^{3a} represents C₁₋₄ alkyl (optionally substituted and/or terminated with one or more cyano groups) and Hal is as hereinbefore defined, for example at between -25°C and ambient temperature in the presence of a suitable solvent (e.g. diethyl ether).

Compounds of formula IV may be prepared by reaction of a compound of formula XIX, as hereinbefore defined, with a compound of formula III as hereinbefore defined, for example as described hereinbefore for synthesis of compounds of formula I (process step (a)).

Compounds of formula IV may alternatively be prepared by reaction of a compound of formula XIX, as hereinbefore defined, with a compound of formula XV, as hereinbefore defined, in the presence of 1,1'-carbonyldiimidazole, for example as described hereinbefore for synthesis of compounds of formula I (process step (p)).

Compounds of formula IV in which R² and R³ represent H may alternatively be prepared by reduction of a corresponding compound of formula XXII, 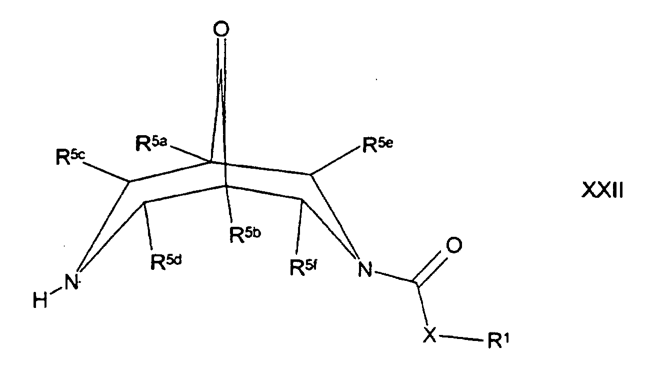 wherein R¹, R^{5a}, R^{5b}, R^{5c}, R^{5d}, R^{5e}, R^{5f} and X are as hereinbefore defined, and in which the bridgehead C=O group may be activated using an appropriate agent, such as tosylhydrazine, for example as described hereinbefore for compounds of formula I (process step (e)).

Compounds of formula IV in which one or more of R^{5c}, R^{5d}, R^{5e} and/or R^{5f} represent C₁₋₃ alkyl may be prepared by reaction of a compound of formula IV in which R^{5c}, R^{5d}, R^{5e} and/or R^{5f} (as appropriate) represent H, with an appropriate alkylating agent (e.g. dimethyl sulfate), for example in the presence of a suitable strong base (e.g. *s*-BuLi), N,N,N',N'-tetramethylethylenediamine and a reaction-inert solvent (e.g. THF).

Compounds of formula V may be prepared in accordance with techniques which are well known to those skilled in the art. For example, compounds of formula V in which:
(1) B represents -CH₂O- and Y represents O may be prepared by reaction of a compound of formula XXIII, 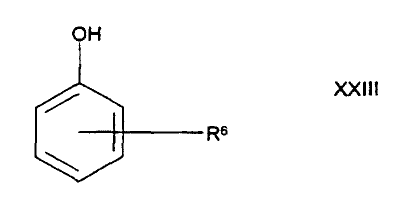 wherein R⁶ is as hereinbefore defined, with a compound of formula XXIV, 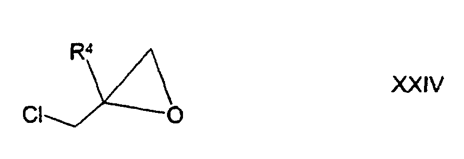 wherein R⁴ is as hereinbefore defined, for example at elevated temperature (e.g. between 60°C and reflux temperature) in the presence of a suitable base (e.g. K₂CO₃ or NaOH) and an appropriate organic solvent (e.g. acetonitrile or toluene/water), or as otherwise described in the prior art;
(2) B represents -CH₂O- and Y represents O may alternatively be prepared by reaction of a compound of formula XXIII, as hereinbefore defined, with a compound of formula XXV, 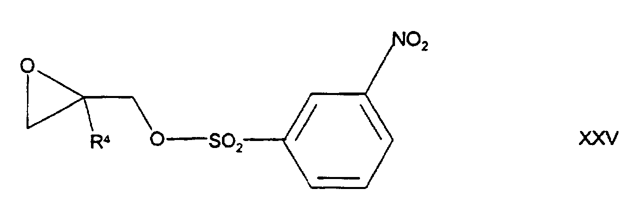 wherein R⁴ is as hereinbefore defined, for example at between room temperature and elevated temperature (e.g. 40°C) in the presence of a suitable base (e.g. K₂CO₃ or potassium ethoxide) and an appropriate organic solvent (e.g. acetonitrile or DMF);
(3) B represents a single bond, Y represents O and R⁴ represents H may be prepared by reduction of a compound of formula XXVI, 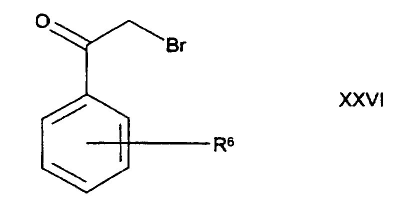 wherein R⁶ is as hereinbefore defined, for example at between -15°C and room temperature in the presence of a suitable reducing agent (e.g. NaBH₄) and an appropriate organic solvent (e.g. THF), followed by an internal displacement reaction of the resultant intermediate, for example at room temperature in the presence of a suitable base (e.g. K₂CO₃) and an appropriate organic solvent (e.g. acetonitrile);
(4) B represents C₁₋₄ alkylene, -(CH₂)ₚN(R²⁶)-, -(CH₂)ₚS(O)₂- or -(CH₂)ₚO- (in which latter three groups p represents 1, 2, 3 or 4) and Y represents O may be prepared by oxidation of a compound of formula XXVII, 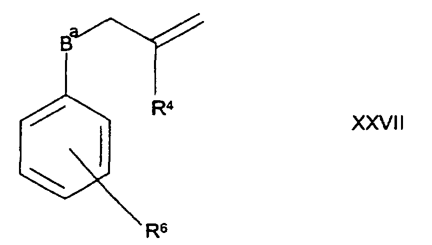 in which B^{a} represents a single bond, C₁₋₃ alkylene, -(CH₂)ₚ₋₁N(R²⁶)-, - (CH₂)ₚ₋₁S(O)₂- or -(CH₂)ₚ₋₁O- (in which latter three groups p represents 1, 2, 3 or 4) and R²⁶ is as hereinbefore defined, in the presence of a suitable oxidising agent (e.g. *m*-chloroperbenzoic acid), for example by refluxing in the presence of a suitable organic solvent (e.g. dichloromethane); or
(5) B represents -(CH₂)ₚO-, Y represents N(R¹⁰) and R¹⁰ represents - S(O)₂R^{15a} or -C(O)OR¹⁹ may be prepared by cyclisation of a compound of formula XXVIIA, 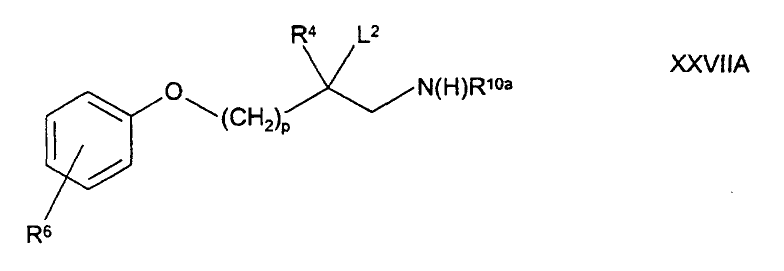 wherein R^{10a} represents -S(O)₂R^{15a} or -C(O)OR¹⁹ and p, R⁴, R⁶, R^{15a}, R¹⁹ and L² are as hereinbefore defined, for example at between 0°C and reflux temperature in the presence of a suitable base (e.g. sodium hydroxide) and an appropriate solvent (e.g. dichloromethane, water, or a mixture thereof) and, if necessary a phase transfer catalyst (such as tetrabutylammonium hydrogensulfate).

Compounds of formula VI may be prepared by standard techniques. For example compounds of formula VI in which:
(1) B represents -(CH₂)ₚO- may be prepared by coupling a compound of formula XXVIII, 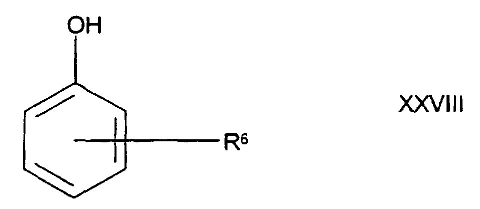 wherein R⁶ is as hereinbefore defined, to a compound of formula XXIX,

   L⁴-(CH₂)ₚ-C(D)(R⁴)-A-L² XXIX

   wherein L⁴ represents a suitable leaving group (e.g. Hal) and Hal, p, R⁴, A, D and L² are as hereinbefore defined;
(2) B represents -C(O)N(R²⁶)- may be prepared by coupling a compound of formula XXX, 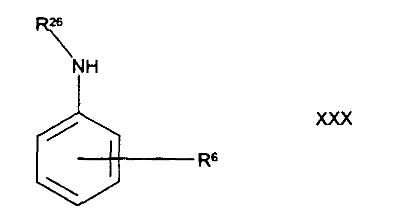 wherein R⁶ and R²⁶ are as hereinbefore defined, to a compound of formula XXXI,

   L⁴-C(O)-C(D)(R⁴)-A-L² XXX XXXI

   wherein L⁴, R⁴, A, D and L² are as hereinbefore defined;
   in both cases, under conditions which are well known to those skilled in the art.

Compounds of formula VI in which A represents C₂-alkylene and D represents OR⁹, in which R⁹ represents optionally substituted C₁₋₆ alkyl, optionally substituted -(CH₂)_{d}-aryl or optionally substituted -(CH₂)_{d}-Het² may alternatively be prepared by reaction of a compound of formula XVI as hereinbefore defined with a compound of formula XXXII, 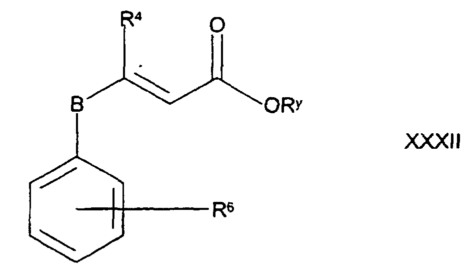 wherein R^{y} represents C₁₋₄ alkyl or aryl (which two groups are optionally substituted with one or more substituents selected from C₁₋₄ alkyl or halo) and R⁴, R⁶ and B are as hereinbefore defined, for example at between ambient temperature (e.g. 25°C) and reflux temperature in the presence of a suitable base (e.g. K₂CO₃) and an appropriate organic solvent (e.g. acetonitrile), followed by conversion of the ester functionality to an L² group (in which L² is as hereinbefore defined), under conditions that are well known to those skilled in the art.

Compounds of formula V and VI in which B represents -(CH₂)ₚS(O)- or - (CH₂)ₚS(O)₂- may be prepared by oxidation of a corresponding compound of formula V or VI (as appropriate) wherein B represents -(CH₂)ₚS-, wherein p is as hereinbefore defined, in the presence of an appropriate amount of a suitable oxidising agent (e.g. *m*-chloroperbenzoic acid) and an appropriate organic solvent.

Compounds of formula VII may be prepared in a similar fashion to compounds of formula I (see, for example, process steps (a), (b) or (c)).

Alternatively, compounds of formula VII in which A represents C₂ alkylene may be prepared by reaction of a corresponding compound of formula IV, as hereinbefore defined with a compound of formula XXXIII, 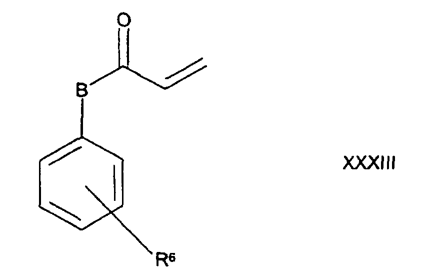 wherein R⁶ and B are as hereinbefore defined, for example a room temperature in the presence of a suitable organic solvent (e.g. ethanol).

Compounds of formula IX may be prepared by reaction of a corresponding compound of formula XXXIIIA, 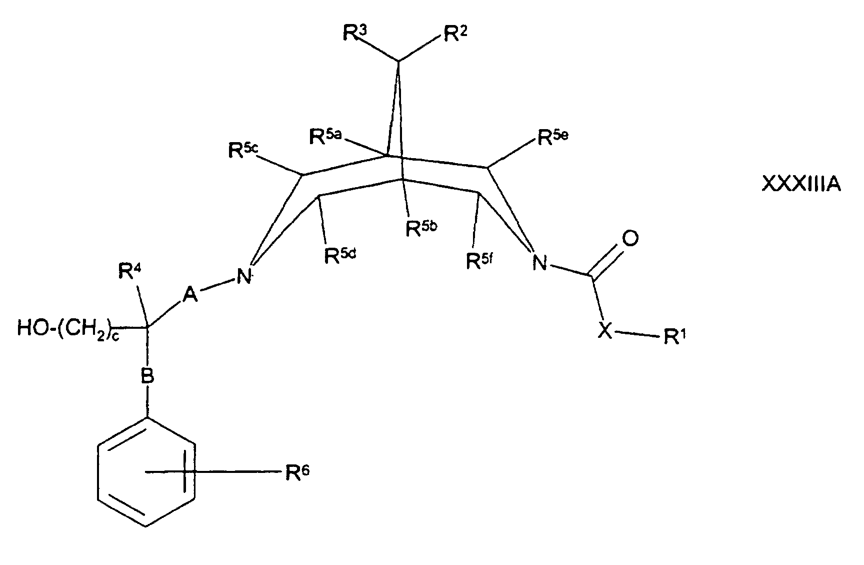 wherein c, R¹, R², R³, R⁴, R^{5a}, R^{5b}, R^{5c}, R^{5d}, R^{5e}, R^{5f}, R⁶, X, A and B are as hereinbefore defined with a compound of formula XXXIV,

R^{y}S(O)₂Cl XXXIV

wherein R^{y} is as hereinbefore defined, for example at between -10 and 25°C in the presence of a suitable solvent (e.g. dichloromethane), followed by reaction with a suitable source of the azide ion (e.g. sodium azide) for example at between ambient and reflux temperature in the presence of an appropriate solvent (e.g. DMF) and a suitable base (e.g. NaHCO₃).

Compounds of formula IX may alternatively be prepared by reaction of a compound of formula IV as hereinbefore defined with a compound of formula XXXIVA, 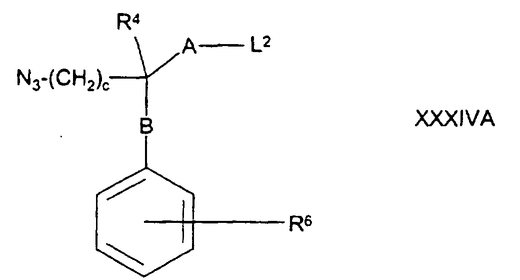 wherein L², R⁴, R⁶, A, B and c are as hereinbefore defined, for example under analogous conditions to those described hereinbefore for preparation of compounds of formula I (process step (c)).

Compounds of formula XIII may be prepared by removing an optionally substituted benzyloxycarbonyl unit from (i.e. deprotecting) a corresponding compound of formula I in which D and R⁴ both represent H and B represents -N(R²⁶)C(O)O(CH₂)-, A represents A^{a} and A^{a} is as hereinbefore defined under conditions which are well known to those skilled in the art.

Compounds of formula XVII may be prepared by replacement of the OH group of a corresponding compound of formula I in which D represents OH with an L² group under conditions that are well known to those skilled in the art.

Compounds of formula XIX in which R² and R³ both represent H may be prepared by reduction of a compound of formula XXXV, 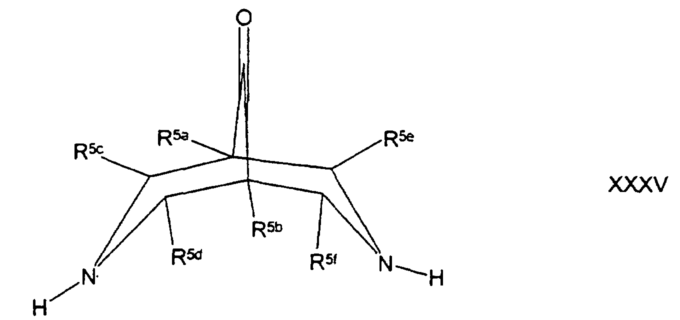 wherein R^{5a}, R^{5b}, R^{5c}, R^{5d}, R^{5e} and R^{5f} are as hereinbefore defined, under appropriate conditions (for example conditions such as those described in respect of the preparation of compounds of formula I (process step (e))).

Compounds of formula XIX in which R² represents OH and R³ represents R^{3a} may be prepared by reaction of a corresponding compound of formula XXXV as hereinbefore defined, with a compound of formula XXI as hereinbefore defined, under appropriate conditions (for example conditions such as those described for the production of compounds of formula II in which R² represents OH and R³ represents R^{3a}).

Compounds of formula XXXIIIA may be prepared in analogous fashion to corresponding compounds of formula I.

Compounds of formula XXXIVA may be prepared in analogous fashion to compounds of formula IX (i.e. from the corresponding alcohol including a - (CH₂)_{c}OH group).

Compounds of formulae VIII, XX, XXII and XXXV (in which, in all cases, R^{5c} and R^{5d} both represent H) may be prepared, advantageously, by reaction of a compound of formula XXXVI, 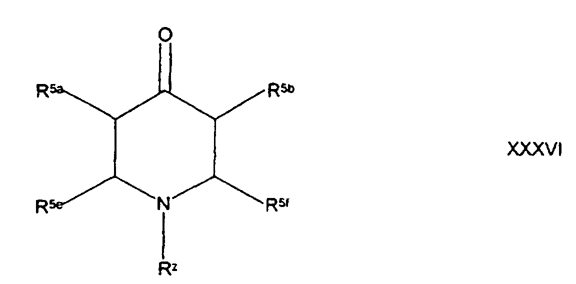 wherein R^{z} represents H or -C(O)XR¹ and R¹, R^{5a}, R^{5b}, R^{5e}, R^{5f} and X are as hereinbefore defined, or a protected derivative thereof, with (as appropriate) either (1) a compound of formula XXXVII, 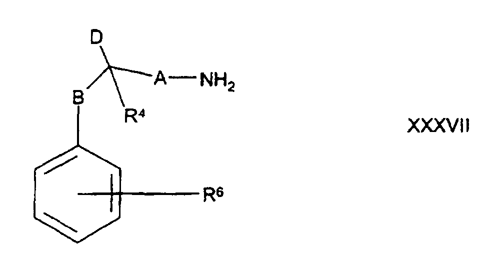 or a protected derivative thereof, wherein R⁴, R⁶, A, B and D are as hereinbefore defined, or (2) NH₃ (or a protected (e.g. benzyl) derivative thereof), in all cases in the presence of a formaldehyde (i.e. an appropriate source of formaldehyde, such as paraformaldehyde or formalin solution).

The formation of compounds of formulae VIII, XX, XXII and XXXV may be carried out in this way for example at between room temperature and reflux (depending upon the concentration of the reactants) in the presence of an appropriate solvent (e.g. ethanol or methanol) and, preferably, in the presence of an organic acid (e.g. a C₁₋₆ carboxylic acid, especially acetic acid).

The skilled person will also appreciate that this process may also be used to prepare compounds of formula I in which R^{5e} and R^{5f} are H, and R^{5c} and/or R^{5d} are other than H, for example by:
(i) reacting a compound of formula XXXVI in which R^{z} represents -C(O)XR¹ and R^{5e} and/or R^{5f} is/are other than H with, for example, benzylamine or a derivative thereof;
(ii) removal of the -C(O)XR¹ unit;
(iii) reaction at the free bispidine nitrogen of the resultant compound with a compound of formula VI as hereinbefore defined;
(iv) removal of the benzyl protecting group; and
(v) reaction at the free bispidine nitrogen of the resultant compound with, for example a compound of formula III or XV as hereinbefore defined,
under conditions well known to those skilled in the art including those described hereinbefore. This reaction will be accompanied by, at some point, conversion of the bridgehead carbonyl functionality to the desired R²/R³ groups.

Compounds of formula XXXVII are well known in the literature or are readily available using known techniques. For example, compounds of formula XXXVII wherein D represents -OH, R⁴ represents H and A represents CH₂ may be prepared by reaction of a compound of formula V in which R⁴ represents H with ammonium hydroxide under conditions which are well known to those skilled in the art.

Compounds of formulae III, VIIIA, X, XI, XIA, XII, XIV, XV, XVI, XVIII, XVIIIA, XXI, XXIII, XXIV, XXV, XXVI, XXVII, XXVIIA, XXVIII, XXIX, XXX, XXXI, XXXII, XXXIII, XXXIV and XXXVI and derivatives thereof, are either commercially available, are known in the literature, or may be obtained either by analogy with the processes described herein, or by conventional synthetic procedures, in accordance with standard techniques, from readily available starting materials using appropriate reagents and reaction conditions.

Substituents on the aryl (e.g. phenyl), and (if appropriate) heterocyclic, group(s) in compounds defined herein may be converted to other substituents using techniques well known to those skilled in the art. For example, nitrobenzene may be reduced to an aminobenzene, hydroxy may be converted to alkoxy, alkoxy may be hydrolysed to hydroxy etc.

The compounds of the invention may be isolated from their reaction mixtures using conventional techniques.

It will be appreciated by those skilled in the art that, in the processes described above, the functional groups of intermediate compounds may be, or may need to be, protected by protecting groups.

Functional groups which it is desirable to protect include hydroxy, amino and carboxylic acid. Suitable protecting groups for hydroxy include trialkylsilyl and diarylalkylsilyl groups (e.g. *tert*-butyldimethylsilyl, *tert*-butyldiphenylsilyl or trimethylsilyl), tetrahydropyranyl and alkylcarbonyloxy groups (e.g. methyl- and ethylcarbonyloxy groups). Suitable protecting groups for amino include benzyl, *tert*-butyloxycarbonyl, 9-fluorenylmethoxycarbonyl or benzyloxycarbonyl. Suitable protecting groups for carboxylic acid include C₁₋₆ alkyl or benzyl esters.

The protection and deprotection of functional groups may take place before or after any of the reaction steps described hereinbefore.

Protecting groups may be removed in accordance with techniques which are well known to those skilled in the art and as described hereinafter.

The use of protecting groups is fully described in "Protective Groups in Organic Chemistry", edited by J W F McOmie, Plenum Press (1973), and "Protective Groups in Organic Synthesis", 2nd edition, T W Greene & P G M Wutz, Wiley-Interscience (1991).

Persons skilled in the art will appreciate that, in order to obtain compounds of the invention in an alternative, and, on some occasions, more convenient, manner, the individual process steps mentioned herein may be performed in a different order, and/or the individual reactions may be performed at a different stage in the overall route (i.e. substituents may be added to and/or chemical transformations performed upon, different intermediates to those associated hereinbefore with a particular reaction). This will depend *inter alia* on factors such as the nature of other functional groups present in a particular substrate, the availability of key intermediates and the protecting group strategy (if any) to be adopted. Clearly, the type of chemistry involved will influence the choice of reagent that is used in the said synthetic steps, the need, and type, of protecting groups that are employed, and the sequence for accomplishing the synthesis.

It will also be appreciated by those skilled in the art that, although certain protected derivatives of compounds of formula I, which may be made prior to a final deprotection stage, may not possess pharmacological activity as such, they may be administered parenterally or orally and thereafter metabolised in the body to form compounds of the invention which are pharmacologically active. Such derivatives may therefore be described as "prodrugs". Moreover, certain compounds of formula I may act as prodrugs of other compounds of formula I.

All prodrugs of compounds of formula I are included within the scope of the invention.

Some of the intermediates referred to hereinbefore are novel. According to a further aspect of the invention there is thus provided: (a) a compound of formula II as hereinbefore defined, or a protected derivative thereof; (b) a compound of formula IV as hereinbefore defined, or a protected derivative thereof; (c) a compound of formula VIII as hereinbefore defined, or a protected derivative thereof; (d) a compound of formula XX as hereinbefore defined, or a protected derivative thereof; and (e) a compound of formula XXII as hereinbefore defined, or a protected derivative thereof.

### Medical and pharmaceutical use

The compounds of the invention are useful because they possess pharmacological activity. They are therefore indicated as pharmaceuticals.

Thus, according to a further aspect of the invention there is provided the compounds of the invention for use as pharmaceuticals.

In particular, the compounds of the invention exhibit myocardial electrophysiological activity, for example as demonstrated in the test described below.

The compounds of the invention are thus expected to be useful in both the prophylaxis and the treatment of arrhythmias, and in particular atrial and ventricular arrhythmias.

The compounds of the invention are thus indicated in the treatment or prophylaxis of cardiac diseases, or in indications related to cardiac diseases, in which arrhythmias are believed to play a major role, including ischaemic heart disease, sudden heart attack, myocardial infarction, heart failure, cardiac surgery and thromboembolic events.

In the treatment of arrhythmias, compounds of the invention have been found to selectively delay cardiac repolarization, thus prolonging the QT interval, and, in particular, to exhibit class III activity. Although compounds of the invention have been found to exhibit class III activity in particular, in the treatment of arrhythmias, their mode(s) of activity is/are not necessarily restricted to this class.

According to a further aspect of the invention, there is provided the use of compound of the invention as active ingredient in the manufacture of a medicament for use in the prophylaxis or the treatment of an arrhythmia.

### Pharmaceutical preparations

The compounds of the invention will normally be administered orally, subcutaneously, intravenously, intraarterially, transdermally, intranasally, by inhalation, or by any other parenteral route, in the form of pharmaceutical preparations comprising the active ingredient either as a free base, a pharmaceutically acceptable ion exchanger or a non-toxic organic or inorganic acid addition salt, in a pharmaceutically acceptable dosage form. Depending upon the disorder and patient to be treated, as well as the route of administration, the compositions may be administered at varying doses.

The compounds of the invention may also be combined with any other drugs useful in the treatment of arrhythmias and/or other cardiovascular disorders.

According to a further aspect of the invention there is thus provided a pharmaceutical formulation including a compound of the invention in admixture with a pharmaceutically acceptable adjuvant, diluent or carrier.

Suitable daily doses of the compounds of the invention in therapeutic treatment of humans are about 0.05 to 5.0 mg/kg body weight at parenteral administration.

The compounds of the invention have the advantage that they are effective against cardiac arrhythmias.

Compounds of the invention may also have the advantage that they may be more efficacious than, be less toxic than, have a broader range of activity (including exhibiting any combination of class I, class II, class III and/or class IV activity (especially class I, class II and/or class IV activity in addition to class III activity)) than, be more potent than, produce fewer side effects (including a lower incidence of proarrhythmias such as *torsades de pointes*) than, be more easily absorbed than, or that they may have other useful pharmacological properties over, compounds known in the prior art.

### Biological Tests

### Test A

### Primary Electrophysiological Effects In Anaesthetised Guinea Pigs

Guinea pigs weighing between 660 an 1100 g were used. The animals were housed for at least one week before the experiment and had free access to food and tap water during that period.

Anaesthesia was induced by an intraperitoneal injection of pentobarbital (40 to 50 mg/kg) and catheters were introduced into one carotid artery (for blood pressure recording and blood sampling) and into one jugular vein (for drug infusions). Needle electrodes were placed on the limbs for recording of ECGs (lead II). A thermistor was placed in the rectum and the animal was placed on a heating pad, set to a rectal temperature of between 37.5 and 38.5°C.

A tracheotomy was performed and the animal was artificially ventilated with room air by use of a small animal ventilator, set to keep blood gases within the normal range for the species. In order to reduce autonomic influences both vagi were cut in the neck, and 0.5 mg/kg of propranolol was given intravenously, 15 minutes before the start of the experiment.

The left ventricular epicardium was exposed by a left-sided thoracotomy, and a custom-designed suction electrode for recording of the monophasic action potential (MAP) was applied to the left ventricular free wall. The electrode was kept in position as long as an acceptable signal could be recorded, otherwise it was moved to a new position. A bipolar electrode for pacing was clipped to the left atrium. Pacing (2 ms duration, twice the diastolic threshold) was performed with a custom-made constant current stimulator. The heart was paced at a frequency just above the normal sinus rate during 1 minute every fifth minute throughout the study.

The blood pressure, the MAP signal and the lead II ECG were recorded on a Mingograph ink-jet recorder (Siemens-Elema, Sweden). All signals were collected (sampling frequency 1000 Hz) on a PC during the last 10 seconds of each pacing sequence and the last 10 seconds of the following minute of sinus rhythm. The signals were processed using a custom-made program developed for acquisition and analysis of physiological signals measured in experimental animals (see Axenborg and Hirsch, Comput. Methods Programs Biomed. **41,** 55 (1993)).

The test procedure consisted of taking two basal control recordings, 5 minutes apart, during both pacing and sinus rhythm. After the second control recording, the first dose of the test substance was infused in a volume of 0.2 mL into the jugular vein catheter for 30 seconds. Three minutes later, pacing was started and a new recording was made. Five minutes after the previous dose, the next dose of test substance was administered. Six to ten consecutive doses were given during each experiment.

### Data analysis

Of the numerous variables measured in this analysis, three were selected as the most important for comparison and selection of active compounds. The three variables selected were the MAP duration at 75 percent repolarization during pacing, the atrio-ventricular (AV) conduction time (defined as the interval between the atrial pace pulse and the start of the ventricular MAP) during pacing, and the heart rate (defined as the RR interval during sinus rhythm). Systolic and diastolic blood pressure were measured in order to judge the haemodynamic status of the anaesthetised animal. Further, the ECG was checked for arrhythmias and/or morphological changes.

The mean of the two control recordings was set to zero and the effects recorded after consecutive doses of test substance were expressed as percentage changes from this value. By plotting these percentage values against the cumulative dose administered before each recording, it was possible to construct dose-response curves. In this way, each experiment generated three dose-response curves, one for MAP duration, one for AV-conduction time and one for the sinus frequency (RR interval). A mean curve of all experiments performed with a test substance was calculated, and potency values were derived from the mean curve. All dose-response curves in these experiments were constructed by linear connection of the data points obtained. The cumulative dose prolonging the MAP duration by 10% from the baseline was used as an index to assess the class III electrophysiological potency of the agent under investigation (D₁₀).

The invention is illustrated by way of the following examples.

### Examples

### General Experimental Procedures

Mass spectra were recorded on a Finnigan MAT TSQ 700 triple quadrupole mass spectrometer equipped with an electrospray interface (FAB-MS) and VG Platform II mass spectrometer equipped with an electrospray interface (LC-MS), a Hewlett Packard model 6890 gas chromatograph connected to a Hewlett-Packard model 5973A mass spectrometer via a Hewlett Packard HP-5-MS GC column, or a Shimadzu QP-5000 GC/mass spectrometer (CI, methane). ¹H NMR and ¹³C NMR measurements were performed on a BRUKER ACP 300 and Varian UNITY plus 400 and 500 spectrometers, operating at ¹H frequencies of 300, 400 and 500 MHz respectively, and at ¹³C frequencies of 75.5, 100.6 and 125.7 MHz respectively. Alternatively, ¹³C NMR measurements were performed on a BRUKER ACE 200 spectrometer at a frequency of 50.3 MHz.

Rotamers may or may not be denoted in spectra depending upon ease of interpretation of spectra. Unless otherwise stated, chemical shifts are given in ppm with the solvent as internal standard.

### Example 1

### tert-Butyl 7-[3-(4-cyanophenoxy)-2-hydroxypropyl]-2,4-dimethyl-3,7-diazabicyclo[3.3.1]nonane-3-carboxylate

### (i) 3,7-Dibenzyl-3,7-diazabicyclo[3.3.1]nonane-9-one

The sub-title compound was prepared according to the procedure described in *J. Org. Chem*., **41** (1976) 1593-1597.

### (ii) 3,7-Dibenzyl-3,7-diazabicyclo[3.3.1]nonane

The sub-title compound was prepared according to the procedure described in *J*. *Org*. *Chem*., **41** (1976) 1593-1597, using 3,7-dibenzyl-3,7-diazabicyclo[3.3.1]nonane-9-one (from step (i) above) in place of N-benzyl-N'-methylbispidone.

### (iii) 3-Benzyl-3,7-diazabicyclo[3.3.1]nonane

A solution of 3,7-dibenzyl-3,7-diazabicyclo[3.3.1]nonane (from step (ii) above; 97 g; 6.4 mmol) in aqueous ethanol (95%) was hydrogenated over 5 % Pd/C at 1 atm. until tlc indicated that the reaction was complete. The catalyst was removed by filtration through a pad of Celite®, and the filtrate concentrated under reduced pressure to give the sub-title compound in quantitative yield.
¹³C NMR in CDCl₃: δ 30.1, 33.4, 36.0, 52.5, 59.6, 64.3, 126.9, 128.3, 128.7, 138.8.

### (iv) tert-Butyl 7-benzyl-3,7-diazabicyclo[3.3.1]nonane-3-carboxylate

Di-*tert*-butyl dicarbonate was added slowly to a solution of 3-benzyl-3,7-diazabicyclo[3.3.1]nonane (from step (iii) above; 60 g; 277 mmol) in THF (600 mL). The reaction was stirred at n until all of the starting material had been consumed (as indicated by tlc). The solvent was then removed under reduced pressure to give a quantitative yield of the sub-title compound.

### (v) tert-Butyl 7-benzyl-2-methyl-3,7-diazabicyclo[3.3.1]nonane-3-carboxylate

N,N,N',N'-Tetramethylethylenediamine (0.98 g; 8.4 mmol) and subsequently *s*-BuLi in cyclohexane (8.46 mL; 1.3 M; 11.0 mmol) was added to a cooled (-70°C), stirred solution of *tert*-butyl 7-benzyl-3,7-diazabicyclo[3.3.1]nonane-3-carboxylate (from step (iv) above; 2.65 g; 8.4 mmol) in THF (17 mL) under an inert atmosphere (N₂). The reaction mixture was then allowed to warm to -40°C, at which temperature it was stirred for 1 h. The temperature was lowered again to -70°C, and a solution of dimethyl sulfate (1.64 g; 13.0 mmol) in THF (5 mL) was added. The temperature was then allowed to reach rt before the solvent was evaporated and the residue partitioned between diethyl ether and water. The organic layer was separated, dried (Na₂SO₄), concentrated and subjected to column chromatography (CH₂Cl₂:MeOH; 40:1) to give the sub-title compound in a 30% yield.

### (vi) tert-Butyl 7-benzyl-2,4-dimethyl-3,7-diazabicyclo[3.3.1]nonane-3-carboxylate

The sub-title compound was prepared in a 65% yield according to the procedure described in step (v) above, using *tert*-butyl 7-benzyl-2-methyl-3,7-diazabicyclo[3.3.1]nonane-3-carboxylate (from step (v) above) in place of *tert*-butyl 7-benzyl-3,7-diazabicyclo[3.3.1]nonane-3-carboxylate.

### (vii) tert-Butyl 2,4-dimethyl-3,7-diazabicyclo[3.3.1]nonane-3-carboxylate

The sub-title compound was prepared in quantitative yield according to the procedure described in step (iii) above, using *tert*-butyl 7-benzyl-2,4-dimethyl-3,7-diazabicyclo[3.3.1]nonane-3-carboxylate (from step (vi) above) in place of 3,7-dibenzyl-3,7-diazabicyclo[3.3.1]nonane.

### (viii) tert-Butyl 7-[3-(4-cyanophenoxy)-2-hydroxypropyl]-2,4-dimethyl-3,7-diazabicyclo[3.3.1]nonane-3-carboxylate

The title compound was prepared in 75 % yield (after purification by column chromatography) according to the procedure described in Example 2(iii) below, using *tert*-butyl 2,4-dimethyl-3,7-diazabicyclo[3.3.1]nonane-3-carboxylate (from step (vi) above) in place of 3-benzyl-6,8-dimethyl-3,7-diazabicyclo[3.3.1]nonane.
FAB-MS: m/z = 430.0 (M + H)⁺
¹³C NMR in CD₃CN: δ 18.75, 21.04, 28.32, 28.57, 35.38, 36.91, 51.37, 53.24, 55.69, 59.31, 61.03, 62.19, 66.18, 71.85, 79.09, 104.32, 116.23, 119.76, 134.83, 156.62, 163.26

### Example 2

### tert-Butyl 7-[3-(4-cyanophenoxy)-2-hydroxypropyl]-6,8-dimethyl-3,7-diazabicyclo[3.3.1]nonane-3-carboxylate

### (i) 4-(2-Oxiranylmethoxy)benzonitrile

Epichlorohydrin (800 mL) and K₂CO₃ (414 g) were added to a stirred solution of *p*-cyanophenol (238 g) in 2.0 L of acetonitrile. The reaction mixture was brought to reflux under an inert atmosphere for 2 h before being filtered whilst still hot. The resulting filtrate was concentrated to give a clear oil. This was crystallized from di-*iso*-propyl ether to give the sub-title compound in a 75 % yield.

### (ii) 3-Benzyl-6,8-dimethyl-3,7-diazabicyclo[3.3.1]nonane

Ethyl acetate (10 mL) saturated with HCl was added to a stirred solution of *tert*-butyl 7-benzyl-2,4-dimethyl-3,7-diazabicyclo[3.3.1]nonane-3-carboxylate (from Example 1(vi) above; 1.04 g; 3.01 mmol) in ethyl acetate (5 mL). The reaction mixture was stirred for 2 h at rt, before the solvent was removed under reduced pressure. The residue was redissolved in EtOH and passed through an ion-exchange resin (Amberlyst® IRA 400), concentrated and then lyophilised to give the sub-title compound in quantitative yield.

### (iii) 3-Benzyl-7-[3-(4-cyanophenoxy)-2-hydroxypropyl]-6,8-dimethyl-3,7-diazabicyclo[3.3.1]nonane; Diastereoisomers 1 and 2

A mixture of from 3-benzyl-6,8-dimethyl-3,7-diazabicyclo[3.3.1]nonane (from step (ii) above; 11.1 g; 45.5 mmol) and 4-(2-oxiranylmethoxy)-benzonitrile (from step (i) above; 7.97 g; 45.5 mmol) in IPA-water (44 mL of 9: 1) was stirred at 60°C for 12 h. The reaction mixture was concentrated under reduced pressure and the residue re-dissolved in CH₂Cl₂ and washed with first brine then water. The organic layer was separated, dried (Na₂SO₄) and concentrated. The crude mixture consisted of 4 diastereoisomers (a mixture of 2 diastereomeric pairs). The diastereomeric pairs were separated by chromatography on silica (DCM with 10% NH₃ satd. MeOH).

### (iv) 3-[3-(4-Cyanophenoxy)-2-hydroxypropyl]-2,4-dimethyl-3,7-diazabicyclo[3.3.1]nonane; Diastereoisomers 1 and 2

The sub-title compound pairs was prepared in quantitative yield according to the procedure described in Example 1(iii), using the diastereomeric pairs of 3-benzyl-7-[3-(4-cyanophenoxy)-2-hydroxypropyl]-6,8-dimethyl-3,7-diazabicyclo[3.3.1]nonane (pair from step (iii) above) in place of 3,7-dibenzyl-3,7-diazabicyclo[3.3.1]nonane.

### (v) tert-Butyl 7-[3-(4-cyanophenoxy)-2-hydroxypropyl]-6,8-dimethyl-3,7-diazabicyclo[3.3.1]nonane-3-carboxylate; Diastereoisomers 1

The title compound was prepared in 50 % yield according to the procedure described in Example 1(iv), using 3-[3-(4-cyanophenoxy)-2-hydroxypropyl]-2,4-dimethyl-3,7-diazabicyclo[3.3.1]nonane (Diastereomers 1 from step (iv) above) in place of 3-benzyl-3,7-diazabicyclo[3.3.1]nonane.
FAB-MS: m/z = 429.9 (M + H)⁺
¹³C NMR in CDCl₃: δ 11.13, 19.52, 28.15, 28.49, 34.46, 35.89, 44.80, 48.86, 53.27, 54.98, 61.15, 70.04, 70.72, 79.65, 103.87, 115.33, 119.15, 133.81, 156.23, 162.15

### (vi) tert-Butyl 7-[3-(4-cyanophenoxy)-2-hydroxypropyl]-6,8-dimethyl-3,7-diazabicyclo[3.3.1]nonane-3-carboxylate; Diastereoisomers 2

The title compound was prepared in 50% yield according to the procedure described in Example 1(iv), using 3-[3-(4-cyanophenoxy)-2-hydroxypropyl]-2,4-dimethyl-3,7-diazabicyclo[3.3.1]nonane (Diastereoisomers 2 from step (iv) above) in place of 3-benzyl-3,7-diazabicyclo[3.3.1]nonane.
FAB-MS: m/z = 429.7 (M + H)⁺
¹³C NMR in CDCl₃: δ 10.10, 19.68, 27.67, 28.69, 34.73, 36.01, 44.99, 48.92, 51.25, 52.56, 54.72, 65.01, 71.09, 79.49,103.97, 115.44, 119.24, 133.88, 155.56, 162.26

### Example 3

### tert-Butyl 7-[3-(4-cyanophenoxy)-2-hydroxypropyl]-6-methyl-3,7-diazabicyclo[3.3.1]nonane-3-carboxylate

### (i) 3-Benzyl-6-methyl-3,7-diazabicyclo[3.3.1]nonane

The sub-title compound was prepared according to the procedure described in Example 2(ii) above, using tert-butyl 7-benzyl-2-methyl-3,7-diazabicyclo[3.3.1]nonane-3-carboxylate (Example 1(v) above) in place of *tert*-butyl 7-benzyl-2,4-dimethyl-3,7-diazabicyclo[3.3.1]nonane-3-carboxylate.

### (ii) 3-Benzyl-7-[3-(4-cyanophenoxy)-2-hydroxypropyl]-6-methyl-3,7-diazabicyclo[3.3.1]nonane

The sub-title compound was prepared according to the procedure described in Example 2(iii) above, using 3-benzyl-6-methyl-3,7-diazabicyclo[3.3.1]nonane (from step (i) above) in place of 3-benzyl-6,8-dimethyl-3,7-diazabicyclo[3.3.1]nonane.

### (iii) 3-[3-(4-Cyanophenoxy)-2-hydroxypropyl]-2-methyl-3,7-diazabicyclo[3.3.1]nonane

The sub-title compound was prepared according to the procedure described in Example 1(iii) above, using 3-benzyl-7-[3-(4-cyanophenoxy)-2-hydroxypropyl]-6-methyl-3,7-diazabicyclo[3.3.1]nonane (from step (ii) above) in place of 3,7-dibenzyl-3,7-diazabicyclo[3.3.1]nonane.

### (iv) tert-Butyl 7-[3-(4-cyanophenoxy)-2-hydroxypropyl]-6-methyl-3,7-diazabicyclo[3.3.1]nonane-3-carboxylate

The title compound was prepared according to the procedure described in Example 1(iv) above, using 3-[3-(4-cyanophenoxy)-2-hydroxypropyl]-2-methyl-3,7-diazabicyclo[3.3.1]nonane (from step (iii) above) in place of 3-benzyl-3,7-diazabicyclo[3.3.1]nonane.
FAB-MS: m/z = 415.8 (M + H)⁺
¹³C NMR in CDCl₃: δ 19.45, 28.55, 29.31, 33.77, 36.13, 44.54, 47.65, 57.32, 58.77, 59.84, 60.71, 62.28, 64.98, 70.48, 79.53, 103.96, 115.38, 119.17, 133.86, 155.42, 162.08

### Example 4

### tert-Butyl 7-[(2S)-3-(4-cyanophenoxy)-2-hydroxypropyl]-6,8-dimethyl-3,7-diazabicyclo[3.3.1]nonane-3-carboxylate

### (i) 4-[(2S)-Oxiranylmethoxy]benzonitrile

The sub-title compound was prepared in a 90% yield according to the procedure described in Example 2(i) above, but using (*R*)-(-)-epichlorohydrin.
¹³C NMR in CDCl₃: δ 44.4, 49.7, 69.0, 104.6, 115.3, 119.0, 134.0, 161.6.

### (ii) 3-Benzyl-7-[(2S)-3-(4-cyanophenoxy)-2-hydroxypropyl]-6,8-dimethyl-3,7-diazabicyclo[3.3.1]nonane; Diastereoisomers 1 and 2

The sub-title compound was prepared according to the procedure described in Example 2(iii) above, using 4-[(2*S*)-oxiranylmethoxy]benzonitrile (from step (i) above) in place of 4-(2-oxiranylmethoxy)benzonitrile, giving a pair of diastereoisomers. the diastereoisomers were separated by column chromatography on silica (DCM and 10 % NH₃ satd. MeOH).

### (iii) 3-[(2S)-3-(4-Cyanophenoxy)-2-hydroxypropyl]-2,4-dimethyl-3,7-diaza bicyclo[3,3,1]nonane; Diastereoisomers 1 and 2

The sub-title compounds were prepared according to the procedure described in Example 3(iii) above, using 3-benzyl-7-[(2*S*)-3-(4-cyanophenoxy)-2-hydroxypropyl]-6,8-dimethyl-3,7-diazabicyclo[3.3.1]-nonane (diastereoisomers 1 and 2 from step (ii) above) in place of 3-benzyl-7-[3-(4-cyanophenoxy)-2-hydroxypropyl]-6-methyl-3,7-diazabicyclo[3.3.1]nonane.

### (iv) tert-Butyl 7-[(2S)-3-(4-cyanophenoxy)-2-hydroxypropyl]-6,8-dimethyl-3,7-diazabicyclo[3.3.1]nonane-3-carboxylate; Diastereoisomers 1

Prepared according to the procedure described in Example 1(iv) above, using 3-[(2*S*)-3-(4-cyanophenoxy)-2-hydroxypropyl]-2-methyl-3,7-diazabicyclo[3.3.1]nonane (distereoisomers 1 from step (iii) above) in place of 3-benzyl-3,7-diazabicyclo[3.3.1]nonane.
ESI-MS: m/z = 429.9 (M + H)⁺
¹³C NMR in CDCl₃: δ 10.09, 19.66, 27.67, 28.69, 34.72, 36.03, 44.99, 48.91, 51.24, 52.55, 54.71, 65.01, 71.09. 79.48, 103.96, 115.44, 119.23, 133.87, 155.56, 162.26

### (v) tert-Butyl 7-[(2S)-3-(4-cyanophenoxy)-2-hydroxypropyl]-6,8-dimethyl-3,7-diazabicyclo[3.3.1]nonane-3-carboxylate; Diastereoisomers 2

Prepared according to the procedure described in Example 1(iv) above, using 3-[(2*S*)-3-(4-cyanophenoxy)-2-hydroxypropyl]-2-methyl-3,7-diazabicyclo[3.3.1]nonane (distereoisomers 2 from step (iii) above) in place of 3-benzyl-3,7-diazabicyclo[3.3.1]nonane.
ESI-MS: m/z = 429.8 (M + H)⁺
¹³C NMR in CDCl₃: δ 11.22, 19.59, 27.33, 28.57, 34.54, 35.98, 44.90, 48.94, 53.35, 55.14, 61.29, 70.16, 70.76, 79.75, 103.97, 115.37, 119.23, 133.90, 155.51, 162.20

### Example 5

The compounds of the above Examples 1 to 4 were tested in Test A above and were all found to exhibit D₁₀ values of more than 6.0.

### Abbreviations

- AcOH =: acetic acid
- aq. =: aqueous
- atm. =: atmospheres
- Bu =: butyl
- DMF =: dimethylformamide
- EI =: electron ionisation
- Et =: ethyl
- EtOAc =: ethyl acetate
- EtOH =: ethanol
- ESI =: electron spray interface
- FAB =: fast atom bombardment
- h =: hours
- IPA =: *iso*-propanol
- LC =: liquid chromatography
- HPLC =: high performance liquid chromatography
- Me =: methyl
- MeCN =: acetonitrile
- MeOH =: methanol
- min. =: minutes
- MS =: mass spectroscopy
- NADPH =: nicotinamide adenine dinucleotide phosphate, reduced form
- NMR =: nuclear magnetic resonance
- Pd/C =: palladium on carbon
- rt. =: room temperature
- sat. =: saturated
- THF =: tetrahydrofuran
- t.l.c. =: thin layer chromatography

Prefixes *n*, *s*, *i* and *t* have their usual meanings: normal, iso, secondary and tertiary.

## Claims

1. A compound of formula I, 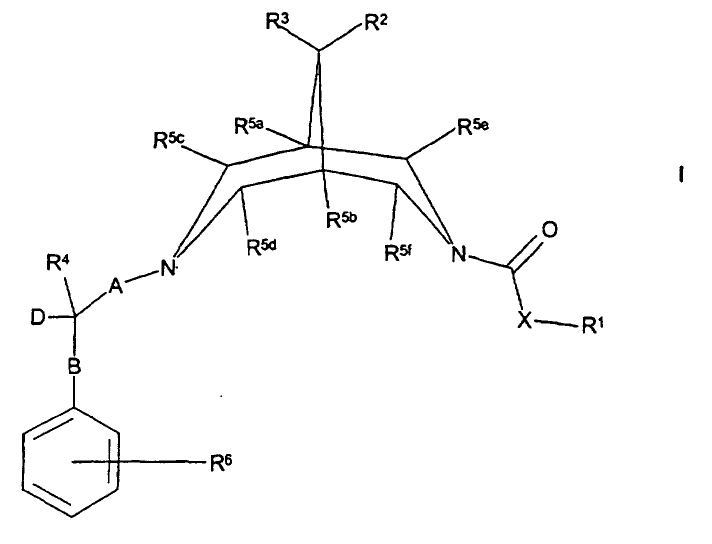 wherein
R¹ represents C₁₋₁₂ alkyl, -(CH₂)ₐ-aryl, or -(CH₂)ₐ-Het¹ (all of which are optionally substituted and/or terminated (as appropriate) by one or more substituents selected from -OH, halo, cyano, nitro, C₁₋₄ alkyl and/or C₁₋₄ alkoxy);
a represents 0, 1, 2, 3, or 4;
Het¹ represents a five to ten-membered heterocyclic ring containing one or more heteroatoms selected from oxygen, nitrogen and/or sulfur, and
which also optionally includes one or more =O substituents;
X represents O or S;
R^{5a}, R^{5b}, R^{5c}, R^{5d}, R^{5e} and R^{5f} independently represent H or C₁₋₃ alkyl;
R² and R³ independently represent H, C₁₋₄ alkyl (optionally substituted and/or terminated with one or more nitro or cyano groups), OR⁷, N(R^{7a})R^{7b}, OC(O)R⁸ or together form -O-(CH₂)₂-O-, -(CH₂)₃-, -(CH₂)₄- or -(CH₂)₅-;
R⁷ and R⁸ independently represent H, C₁₋₆ alkyl or -(CH₂)_{b}-aryl (which latter two groups are optionally substituted and/or terminated by one or more substituents selected from -OH, halo, cyano, nitro, C₁₋₄ alkyl and/or C₁₋₄ alkoxy);
R^{7a} and R^{7b} independently represent H or C₁₋₆ alkyl;
b represents 0, 1, 2, 3 or 4;
R⁴ represents H or C₁₋₆ alkyl;
D represents H, C₁₋₄ alkyl, -OR⁹, or -(CH₂)_{c}N(R¹⁰)(R¹¹);
R⁹ represents H, C₁₋₆ alkyl, -C(O)R¹², -(CH₂)_{d}-aryl or -(CH₂)_{d}-Het² (which latter three groups are optionally substituted by one or more substituents selected from -OH, halo, cyano, nitro, C₁₋₄ alkyl, C₁₋₄ alkoxy, C(O)R¹³, C(O)OR¹⁴ and/or -N(H)S(O)ₑR¹⁵);
R¹⁰ represents H, C₁₋₆ alkyl, -(CH₂)_{f}-aryl, -C(NH)NH₂, -S(O)₂R^{15a}, -[C(O)]_{g}N(R¹⁶)(R¹⁷), -C(O)R¹⁸ or -C(O)OR¹⁹;
e represents 0, 1 or 2;
g represent 1 or 2;
R¹¹ represents H, C₁₋₆ alkyl, -C(O)R²⁰ or -(CH₂)ₕ-aryl (which latter group is optionally substituted and/or terminated (as appropriate) by one or more substituents selected from -OH, cyano, halo, amino, nitro, C₁₋₆ alkyl and/or C₁₋₆ alkoxy);
R¹², R¹³, R¹⁴, R¹⁶, R¹⁷, R¹⁸, R¹⁹ and R²⁰ independently represent H, C₁₋₆ alkyl, Het³ or -(CH₂)ⱼ-aryl (which latter three groups are optionally substituted and/or terminated (as appropriate) by one or more substituents selected from -OH, cyano, halo, amino, nitro, C₁₋₆ alkyl and/or C₁₋₆ alkoxy);
R¹⁵ and R^{15a} independently represent C₁₋₆ alkyl, aryl or -(CH₂)ₖ-aryl (all of which are all optionally substituted and/or terminated (as appropriate) by one or more substituents chosen from halo, nitro, C₁₋₆ alkyl and/or C₁₋₆ alkoxy);
c, d, f, h, j and k independently represent 0, 1, 2, 3 or 4;
Het² and Het³ independently represent five to ten-membered heterocyclic rings containing one or more heteroatoms selected from oxygen, nitrogen and/or sulfur, and which also optionally includes one or more =O substituents;
R⁶ represents H or one or more substituents selected from -OH, cyano, halo, amino, nitro, C₁₋₆ alkyl (optionally terminated by N(H)C(O)OR^{20a}), C₁₋₆ alkoxy, -C(O)N(H)R²¹, -NHC(O)N(H)R²², -N(H)S(O)₂R²³ and/or - OS(O)₂R²⁴;
R²¹ and R²² independently represent H or C₁₋₆ alkyl;
R^{20a}, R²³ and R²⁴ independently represent C₁₋₆ alkyl;
A represents a single bond, C₁₋₆ alkylene, -N(R²⁵)(CH₂)ₘ-, -O(CH₂)ₘ- or -(CH₂)ₘC(H)(OR²⁵)(CH₂)ₙ- (in which latter three groups, the -(CH₂)ₘ-group is attached to the bispidine nitrogen atom and which latter four groups are optionally substituted by one or more -OH groups);
B represents a single bond, C₁₋₄ alkylene, -(CH₂)ₚN(R²⁶)-, -(CH₂)ₚS(O)_{q}-, - (CH₂)ₚO- (in which three latter groups, the -(CH₂)ₚ- group is attached to the carbon atom bearing D and R⁴), -C(O)N(R²⁶)- (in which latter group, the -C(O)- group is attached to the carbon atom bearing D and R⁴), -N(R²⁶)C(O)O(CH₂)ₚ- or -N(R²⁶)(CH₂)ₚ- (in which latter two groups, the N(R²⁶) group is attached to the carbon atom bearing D and R⁴);
m, n and p independently represent 0, 1, 2, 3 or 4;
q represents 0, 1 or 2;
R²⁵ represents H, C₁₋₆ alkyl or C(O)R²⁷;
R²⁶ represents H or C₁₋₆ alkyl;
R²⁷ represents H, C₁₋₆ alkyl, Het⁴ or -(CH₂)ᵣ-aryl (which latter two groups are optionally substituted and/or terminated (as appropriate) by one or more substituents selected from -OH, cyano, halo, amino, nitro, C₁₋₆ alkyl and/or C₁₋₆ alkoxy);
Het⁴ represents a five to ten-membered heterocyclic ring containing one or more heteroatoms selected from oxygen, nitrogen and/or sulfur, and which also optionally includes one or more =O substituents;
r represents 0, 1, 2, 3 or 4;
or a salt, solvate or prodrug thereof;
provided that:
(a) R^{5a}, R^{5b}, R^{5c}, R^{5d}, R^{5e} and R^{5f} do not all simultaneously represent H;
(b) R^{5a} and R^{5b} do not represent C₁₋₃ alkyl when R^{5c}, R^{5d}, R^{5e} and R^{5f} all represent H; and
(c) when D represents -OH or -(CH₂)_{c}N(R¹⁰)R¹¹ in which c represents 0, then:-
(i) A does not represent -N(R²⁵)(CH₂)ₘ-, -O(CH₂)ₘ- or -(CH₂)ₘC(H)(OR²⁵)(CH₂)ₙ- (in which n is 0); and/or
(ii) p does not represent 0 when B represents -(CH₂)ₚN(R²⁶)-, -(CH₂)ₚS(O)_{q}- or -(CH₂)ₚO-.

2. A compound as claimed in Claim 1, wherein R' represents optionally substituted -(CH₂)ₐ-phenyl, in which a is 0, 1, 2 or 3, or optionally substituted, optionally unsaturated, linear, branched or cyclic, C₁₋₈ alkyl (which latter group may also be interrupted by an oxygen atom).

3. A compound as claimed in Claim 1 or Claim 2, wherein R² represents H.

4. A compound as claimed in any one of the preceding claims, wherein R³ represents H.

5. A compound as claimed in any one of the preceding claims, wherein R⁴ represents H or C₁₋₃ alkyl.

6. A compound as claimed in any one of the preceding claims, wherein R^{5a} and R^{5b} either both represent H or both represent methyl.

7. A compound as claimed in any one of the preceding claims, wherein R^{5c}, R^{5d}, R^{5e} and R^{5f} independently represent H or C₁₋₂ alkyl.

8. A compound as claimed in any one of the preceding claims, wherein R⁶ represents one or more substituents selected from C₁₋₆ alkyl (which alkyl group is optionally terminated by a N(H)C(O)OR^{20a} group (in which R^{20a} represents C₁₋₅ alkyl)), cyano, nitro, amino, C(O)N(H)R²¹ and/or - N(H)S(O)₂R²³.

9. A compound as claimed in any one of the preceding claims, wherein X represents O.

10. A compound as claimed in any one of the preceding claims, wherein A represents a single bond or linear, or branched, C₁₋₄ alkylene (which group is also optionally interrupted by O).

11. A compound as claimed in any one of the preceding claims, wherein B represents a single bond, C₁₋₄ alkylene, -(CH₂)ₚO- or -(CH₂)ₚN(R²⁶)- (in which latter two cases p is 1, 2 or 3).

12. A compound as claimed in any one of the preceding claims, wherein D represents H, OR⁹ (in which R⁹ represents H, C₁₋₃ alkyl or optionally substituted phenyl) or N(H)R¹⁰ (in which R¹⁰ represents H or C₁₋₄ alkyl).

13. A pharmaceutical formulation including a compound as defined in any one of Claims 1 to 12 in admixture with a pharmaceutically-acceptable adjuvant, diluent or carrier.

14. A compound as defined in any one of Claims 1 to 12 for use as a pharmaceutical.

15. A compound as defined in any one of Claims 1 to 12 for use in the prophylaxis or the treatment of an arrhythmia.

16. The use of a compound as defined in any of one Claims 1 to 12 as active ingredient in the manufacture of a medicament for use in the prophylaxis or the treatment of an arrhythmia.

17. The use as claimed in Claim 16, wherein the arrhythmia is an atrial or a ventricular arrhythmia.

18. A process for the preparation of a compound of formula I as defined in Claim 1 which comprises:
(a) reaction of a compound of formula II, 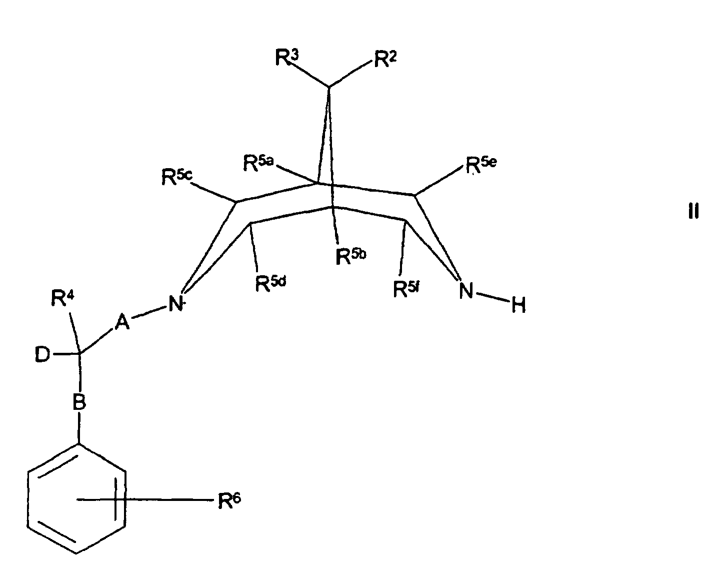 wherein R², R³, R⁴, R^{5a}, R^{5b}, R^{5c}, R^{5d}, R^{5e}, R^{5f}, R⁶, A, B and D are as defined in Claim 1 with a compound of formula III,
R¹XC(O)L¹ III
wherein L' represents a leaving group and R¹ and X are as defined in Claim 1;
(b) for compounds of formula I in which A represents CH₂ and D represents - OH or -N(H)R¹⁰, wherein R¹⁰ is as defined in Claim 1, reaction of a compound of formula IV, 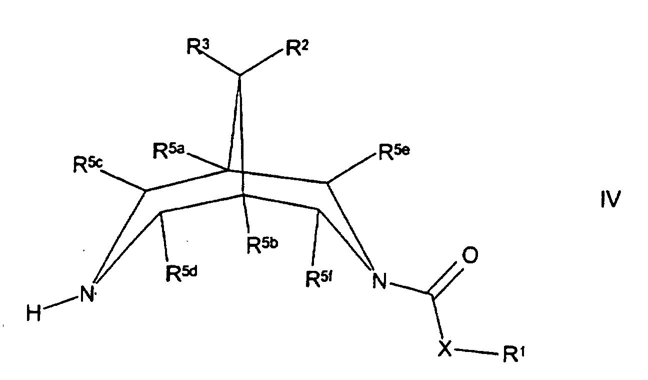 wherein R¹, R², R³, R^{5a}, R^{5b}, R^{5c}, R^{5d}, R^{5e}, R^{5f} and X are as defined in Claim 1, with a compound of formula V, 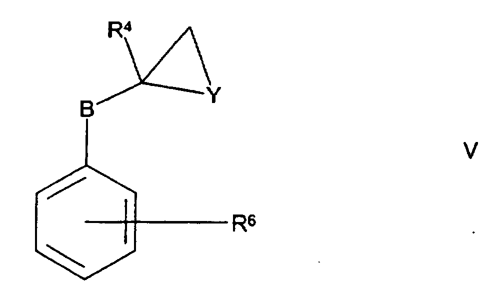 wherein Y represents O or N(R¹⁰) and R⁴, R⁶, R¹⁰ and B are as defined in Claim 1;
(c) reaction of a compound of formula IV, as defined above, with a compound of formula VI, 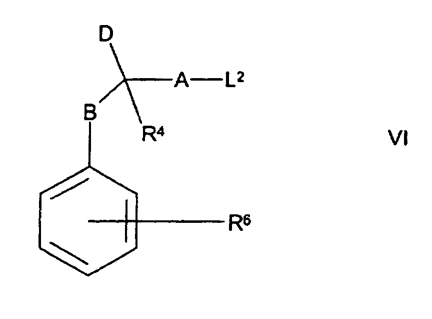 wherein L² represents a leaving group and R⁴, R⁶, A, B and D are as defined in Claim 1;
(d) for compounds of formula I in which D represents H or OH and R⁴ represents H, reduction of a compound of formula VII, 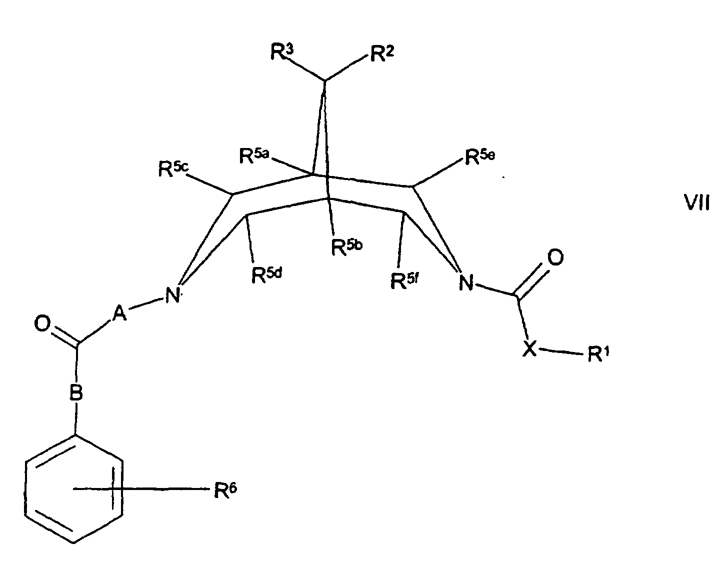 wherein R¹, R², R³, R^{5a}, R^{5b}, R^{5c}, R^{5d}, R^{5e}, R^{5f}, R⁶, A, B and X are as defined in Claim 1;
(e) for compounds of formula I in which one of R² and R³ represents H or OH and the other represents H, reduction of a corresponding compound of formula VIII, 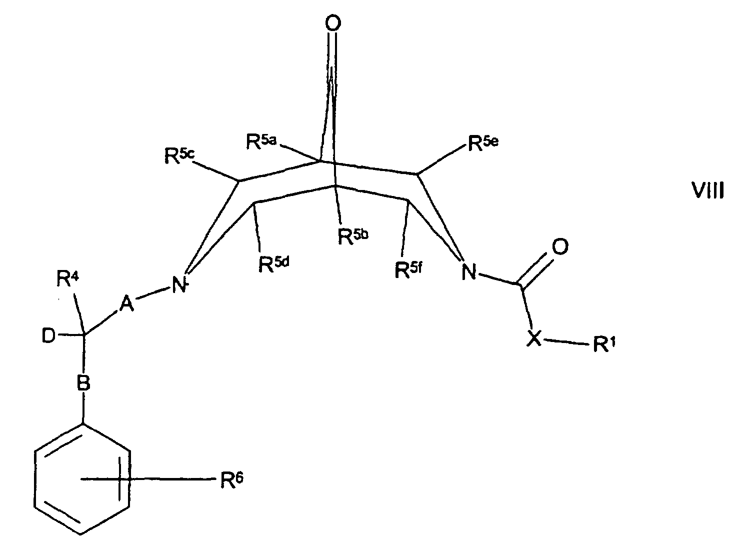 wherein R¹, R⁴, R^{5a}, R^{5b}, R^{5c}, R^{5d}, R^{5e}, R^{5f}, R⁶, A, B, D and X are as defined in Claim 1;
(f) for compounds of formula I in which R² and/or R³ represent OC(O)R⁸ and R⁸ is as defined in Claim 1, coupling of a corresponding compound of formula I in which R² and/or R³ (as appropriate) represent OH and a compound of formula VIIIA,
R⁸CO₂H VIIIA
wherein R⁸ is as defined in Claim 1;
(g) for compounds of formula I in which D represents -(CH₂)_{c}NH₂, reduction of a corresponding compound of formula IX, 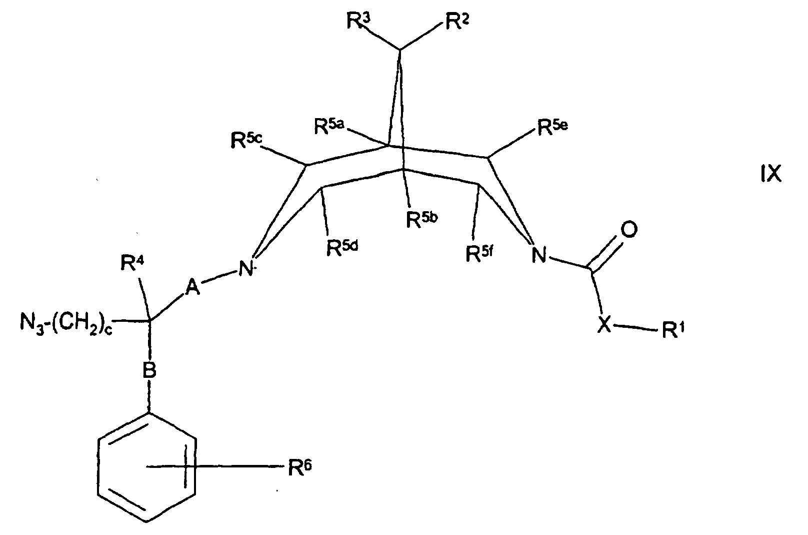 wherein c, R¹, R², R³, R⁴, R^{5a}, R^{5b}, R^{5c}, R^{5d}, R^{5e}, R^{5f}, R⁶, A, B and X are as defined in Claim 1;
(h) for compounds of formula I in which D represents -N(R¹¹)C(O)NH(R¹⁷), in which R¹¹ and R¹⁷ are as defined in Claim 1, except that R¹¹ does not represent C(O)R²⁰, reaction of a corresponding compound of formula I in which D represents -N(R¹¹)H, in which R¹¹ is as defined in Claim 1 except that is does not represent C(O)R²⁰ in which R²⁰ is as defined in Claim 1, with a compound of formula X,
R¹⁷N=C=O X
wherein R¹⁷ is as defined in Claim 1;
(i) for compounds of formula I in which D represents -N(H)[C(O)]₂NH₂, reaction of a corresponding compound of formula I in which D represents - NH₂ with oxalic acid diamide;
(j) for compounds of formula I in which D represents -N(R¹¹)C(O)R¹⁸, in which R¹¹ and R¹⁸ are as defined in Claim 1, except that R¹¹ does not represent C(O)R²⁰, reaction of a corresponding compound of formula I in which D represents -N(R¹¹)H, in which R¹¹ is as defined in Claim 1 except that it does not represent C(O)R²⁰, with a compound of formula XI,
R¹⁸C(O)R^{x} XI
wherein R^{x} represents a suitable leaving group and R¹⁸ is as defined in Claim 1;
(k) for compounds of formula I in which D represents -N(H)R¹⁰ and R¹⁰ is as defined in Claim 1 except that it does not represent H or -C(NH)NH₂, reaction of a corresponding compound of formula I wherein D represents - NH₂ with a compound of formula XIA,
R^{10a}L¹ XIA
wherein R^{10a} represents R¹⁰ as defined in Claim 1, except that it does not represent H or -C(NH)NH₂ and L¹ is as defined above;
(l) for compounds of formula I which are bispidine-nitrogen N-oxide derivatives, oxidation of the corresponding bispidine nitrogen of a corresponding compound of formula I;
(m) for compounds of formula I which are C₁₋₄ alkyl quaternary ammonium salt derivatives, in which the alkyl group is attached to a bispidine nitrogen, reaction, at the bispidine nitrogen, of a corresponding compound of formula I with a compound of formula XII,
R^{a}Hal XII
wherein R^{a} represents C₁₋₄ alkyl and Hal represents Cl, Br or I;
(n) for compounds of formula I in which D and R⁴ both represent H, A represents C₁₋₆ alkylene, B represents -N(R²⁶)(CH₂)ₚ- and R²⁶ and p are as defined in Claim 1, reaction of a compound of formula XIII, 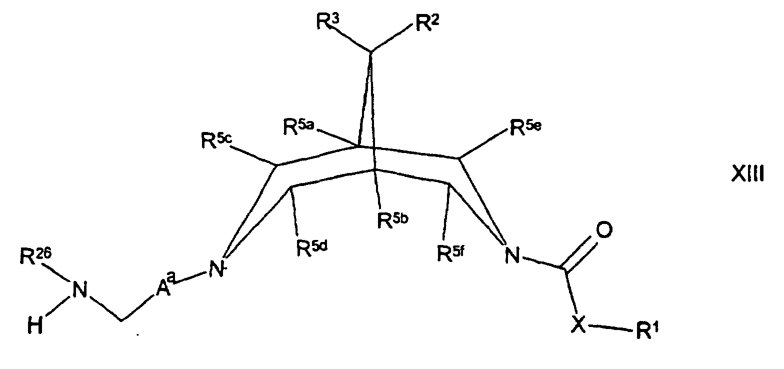 wherein A^{a} represents C₁₋₆ alkylene and R¹, R², R³, R^{5a}, R^{5b}, R^{5c}, R^{5d}, R^{5e}, R^{5f}, R²⁶ and X are as defined in Claim 1 with a compound of formula XIV, 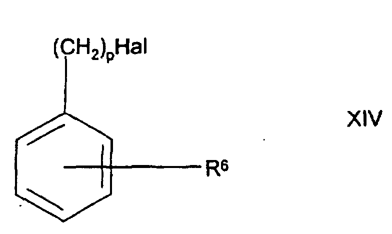 wherein R⁶ and p are as defined in Claim 1 and Hal is defined above;
(o) reaction of a compound of formula II, as defined above, with a compound of formula XV,
R¹XH XV
wherein R¹ and X are as defined in Claim 1, in the presence of 1,1'-carbonyldiimidazole;
(p) for compounds of formula I in which R⁹ represents optionally substituted C₁₋₆ alkyl, optionally substituted -(CH₂)_{d}-aryl or optionally substituted - (CH₂)_{d}-Het², reaction of a corresponding compound of formula I, in which D represents OH with a compound of formula XVI,
R^{9a}OH XVI
wherein R^{9a} represents optionally substituted C₁₋₆ alkyl, optionally substituted -(CH₂)_{d}-aryl or optionally substituted -(CH₂)_{d}-Het², and d and Het² are as defined in Claim 1;
(q) for compounds of formula I in which R⁹ represents optionally substituted C₁₋₆ alkyl, optionally substituted -(CH₂)_{d}-aryl or optionally substituted - (CH₂)_{d}-Het², reaction of a compound of formula XVII, 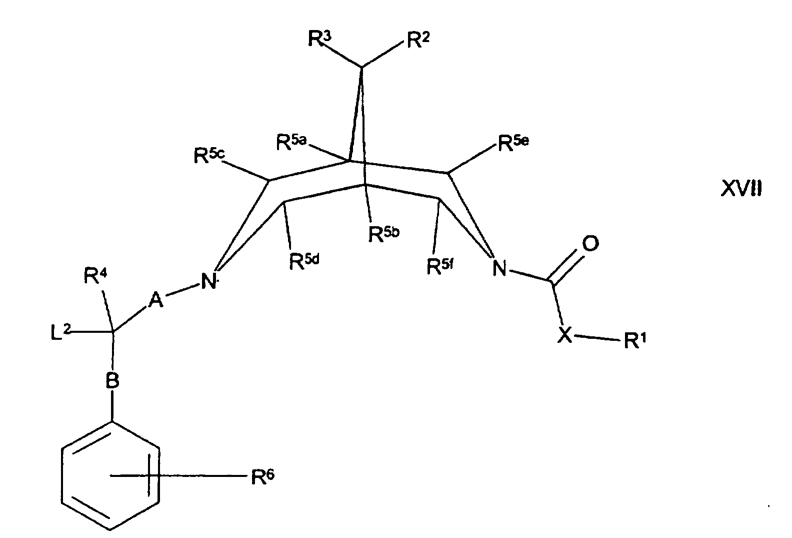 wherein L² is as defined above and R¹, R², R³, R⁴, R^{5a}, R^{5b}, R^{5c}, R^{5d}, R^{5e}, R^{5f}, R⁶, X, A and B are as defined in Claim 1 with a compound of formula XVI as defined above;
(r) for compounds of formula I in which R⁹ represents C(O)R¹² and R¹² is as defined in Claim 1, reaction of a corresponding compound of formula I in which D represents OH with a compound of formula XVIII,
R¹²CO₂H XVIII
wherein R¹² is as defined in Claim 1;
(s) for compounds of formula I in which one or both of R² and R³ represent -N(R^{7a})R^{7b} in which one or both of R^{7a} and R^{7b} represents C₁₋₆ alkyl, alkylation of a corresponding compound of formula I in which R² and/or R³ represent -N(R^{7a})R^{7b} (as appropriate) in which R^{7a} and/or R^{7b} (as appropriate) represent H, using a compound of formula XVIIIA,
R^{7c}L¹ XVIIIA
wherein R^{7c} represents C₁₋₆ alkyl and L¹ is as defined above;
(t) conversion of one R⁶ substituent to another; or
(u) deprotection of a protected derivative of a compound of formula I as defined in Claim 1.

19. A compound of formula II as defined in Claim 18.

20. A compound of formula IV as defined in Claim 18.

21. A compound of formula VIII as defined in Claim 18.

22. A compound of formula XX, 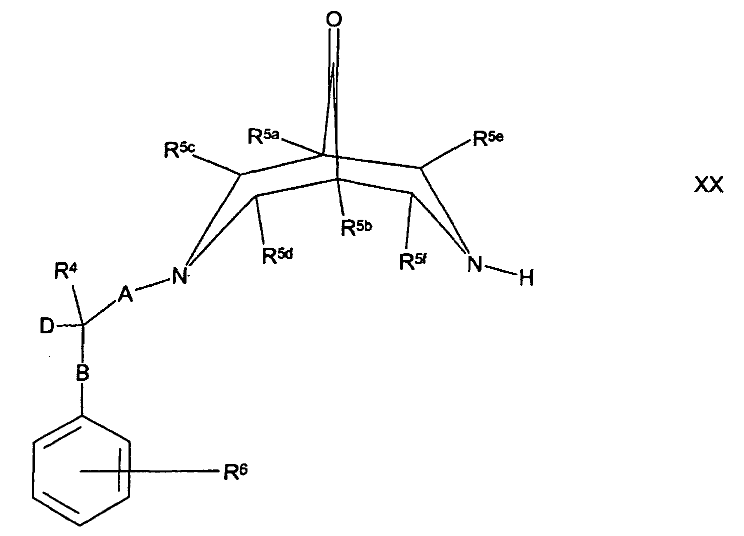 wherein R⁴, R^{5a}, R^{5b}, R^{5c}, R^{5d}, R^{5e}, R^{5f}, R⁶, A, B and D are as defined in Claim 1.

23. A compound of formula XXII, 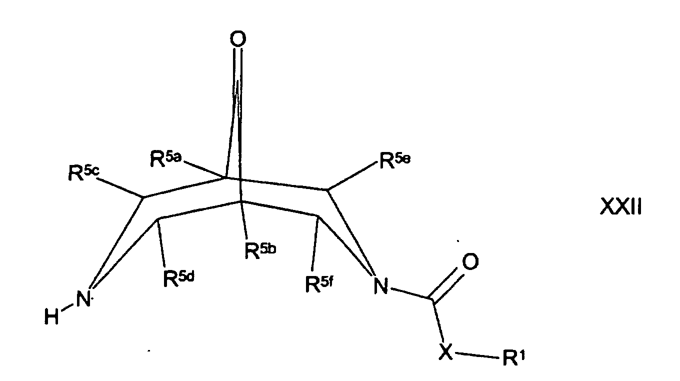 wherein R¹, R^{5a}, R^{5b}, R^{5c}, R^{5d}, R^{5e}, R^{5f} and X are as defined in Claim 1.

24. A process for the preparation of a compound of formula VIII, as defined in Claim 18, a compound of formula XX, as defined in Claim 22, a compound of formula XXII, as defined in Claim 23, or a compound of formula XXXV, 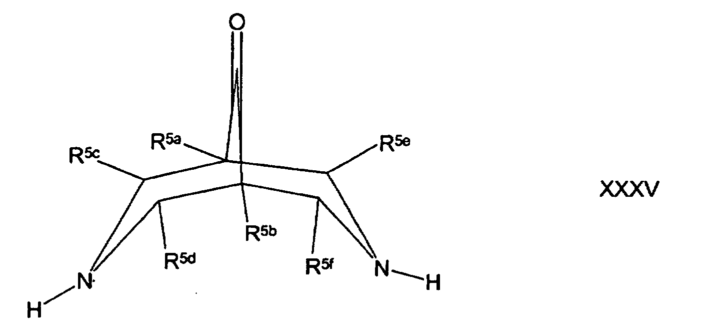 wherein R^{5a} to R^{5f} are as defined in Claim 1, and in which, in all cases, R^{5c} and R^{5d} both represent H, which process comprises reaction of a compound of formula XXXVI, 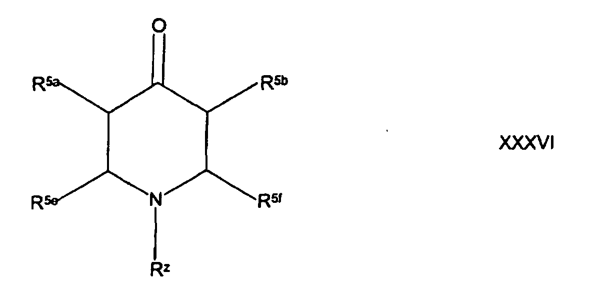 wherein R^{z} represents H or -C(O)XR¹ and R¹, R^{5a}, R^{5b}, R^{5e}, R^{5f} and X are as defined in Claim 1, or a protected derivative thereof, with (as appropriate) either:
(1) a compound of formula XXXVII, 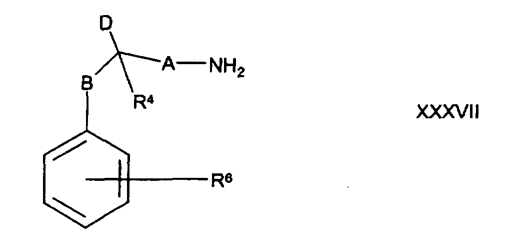 or a protected derivative thereof, wherein R⁴, R⁶, A, B and D are as defined in Claim 1; or
(2) NH₃ (or a protected derivative thereof),
in all cases in the presence of a formaldehyde.

## Patentansprüche

1. Verbindungen der Formel I 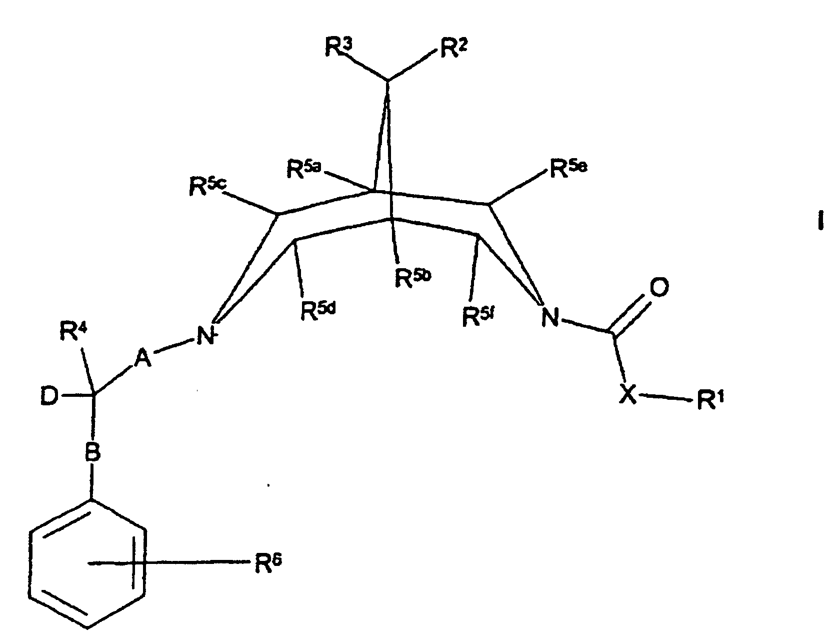 wobei
R¹ für C₁₋₁₂-Alkyl, -(CH₂)ₐ-Aryl oder -(CH₂)ₐ-Het¹ steht (die jeweils gegebenenfalls durch einen oder mehrere Substituenten ausgewählt aus -OH, Halogen, Cyano, Nitro, C₁₋₄-Alkyl und/oder C₁₋₄-Alkoxy (je nach Fall) substituiert und/oder terminiert sind);
a für 0, 1, 2, 3 oder 4 steht;
Het¹ für einen fünf- bis zehngliedrigen heterocyclischen Ring mit einem oder mehreren Heteroatomen ausgewählt aus Sauerstoff, Stickstoff und/oder Schwefel steht, der gegebenenfalls auch einen oder mehrere =O-Substituenten umfaßt, steht;
X für O oder S steht;
R^{5a}, R^{5b}, R^{5c}, R^{5d}, R^{5e} und R^{5f} unabhängig voneinander für H oder C₁₋₃-Alkyl stehen;
R² und R³ unabhängig voneinander für H, C₁₋₄-Alkyl (gegebenenfalls durch eine oder mehrere Nitrooder Cyanogruppen substituiert und/oder terminiert), OR⁷, N(R^{7a})R^{7b} oder OC(O)R⁸ stehen oder zusammen -O-(CH₂)₂-O-, -(CH₂)₃-, -(CH₂)₄- oder -(CH₂)₅- bilden;
R⁷ und R⁸ unabhängig voneinander für H, C₁₋₆-Alkyl oder -(CH₂)_{b}-Aryl stehen (wobei die beiden letztgenannten Gruppen gegebenenfalls durch einen oder mehrere Substituenten ausgewählt aus -OH, Halogen, Cyano, Nitro, C₁₋₄-Alkyl und/oder C₁₋₄-Alkoxy substituiert und/oder terminiert sind);
R^{7a} und R^{7b} unabhängig für H oder C₁₋₆-Alkyl stehen;
b für 0, 1, 2, 3 oder 4 steht;
R⁴ für H oder C₁₋₆-Alkyl steht;
D für H, C₁₋₄-Alkyl, -OR⁹ oder -(CH₂)_{c}N(R¹⁰)(R¹¹) steht;
R⁹ für H, C₁₋₆-Alkyl, -C(O)R¹², -(CH₂)_{d}-Aryl oder - (CH₂)_{d}-Het² steht (wobei die drei letztgenannten Gruppen gegebenenfalls durch einen oder mehrere Substituenten ausgewählt aus -OH, Halogen, Cyano, Nitro, C₁₋₄-Alkyl, C₁₋₄-Alkoxy, C(O)R¹³, C(O)OR¹⁴ und/oder -N(H)S(O)_{c}R¹⁵ substituiert sind);
R¹⁰ für H, C₁₋₆-Alkyl, -(CH₂)_{f}-Aryl, -C(NH)NH₂, -S(O)₂R^{15a}, -[C(O)]_{g}N(R¹⁶)(R¹⁷), -C(O)R¹⁸ oder -C(O)OR¹⁹ steht;
e für 0, 1 oder 2 steht;
g für 1 oder 2 steht;
R¹¹ für H, C₁₋₆-Alkyl, -C(O)R²⁰ oder -(CH₂)ₕ-Aryl steht (wobei die letztgenannte Gruppe gegebenenfalls durch einen oder mehrere Substituenten ausgewählt aus -OH, Cyano, Halogen, Amino, Nitro, C₁₋₆-Alkyl und/oder C₁₋₆-Alkoxy (je nach Fall) substituiert und/oder terminiert ist);
R¹², R¹³, R¹⁴, R¹⁵, R¹⁶, R¹⁷, R¹⁸, R¹⁹ und R²⁰ unabhängig voneinander für H, C₁₋₆-Alkyl, Het³ oder - (CH₂)ⱼ-Aryl stehen (wobei die drei letztgenannten Gruppen gegebenenfalls durch einen oder mehrere Substituenten ausgewählt aus -OH, Cyano, Halogen, Amino, Nitro, C₁₋₆-Alkyl und/oder C₁₋₆-Alkoxy (je nach Fall) substituiert und/oder terminiert sind);
R¹⁵ und R^{15a} unabhängig voneinander für C₁₋₆-Alkyl, Aryl oder -(CH₂)ₖ-Aryl stehen (die jeweils gegebenenfalls durch einen oder mehrere Substituenten ausgewählt aus Halogen, Nitro, C₁₋₆-Alkyl und/oder C₁₋₆-Alkoxy (je nach Fall) substituiert und/oder terminiert sind);
c, d, f, h, j und k unabhängig voneinander für 0, 1, 2, 3 oder 4 stehen;
Het² und Het³ unabhängig voneinander für fünf- bis zehngliedrige heterocyclische Ringe mit einem oder mehreren Heteroatomen ausgewählt aus Sauerstoff, Stickstoff und/oder Schwefel stehen, die gegebenenfalls auch einen oder mehrere =O-Substituenten umfassen;
R⁶ für H oder einen oder mehrere Substituenten ausgewählt aus -OH, Cyano, Halogen, Amino, Nitro, C₁₋₆-Alkyl (gegebenenfalls terminiert durch N(H)C(O)OR^{20a}), C₁₋₆-Alkoxy, -C(O)N(H)R²¹, -NHC(O)N(H)R²², -N(H)S(O)₂R²³ und/oder -OS(O)₂R²⁴ steht;
R²¹ und R²² unabhängig voneinander für H oder C₁₋₆-Alkyl stehen;
R^{20a}, R²³ und R²⁴ unabhängig voneinander für C₁₋₆-Alkyl stehen;
A für eine Einfachbindung, C₁₋₆-Alkylen, -N(R²⁵)(CH₂)ₘ-, -O(CH₂)ₘ- oder -(CH₂)ₘC(H)(OR²⁵) (CH₂)ₙ- steht (wobei in den drei letztgenannten Gruppen die -(CH₂)ₘ-Gruppe an das Bispidin-Stickstoffatom gebunden ist und wobei die vier letztgenannten Gruppen gegebenenfalls durch eine oder mehrere -OH-Gruppen substituiert sind);
B für eine Einzelbindung, C₁₋₄-Alkylen, - (CH₂)ₚN(R²⁶)-, -(CH₂)ₚS(O)_{q}-, -(CH₂)ₚO- (wobei in den drei letztgenannten Gruppen die -(CH₂)ₚ-Gruppe an das Kohlenstoffatom gebunden ist, das D und R⁴ trägt), -C(O)N(R²⁶)- (wobei in der letztgenannten Gruppe die -C(O)-Gruppe an das Kohlenstoffatom gebunden ist, das D und R⁴ trägt), -N(R²⁶)C(O)O(CH₂)ₚ- oder -N(R²⁶)(CH₂)ₚ- steht (wobei in den beiden letztgenannten Gruppen die N(R²⁶)-Gruppe an das Kohlenstoffatom gebunden ist, das D und R⁴ trägt);
m, n und p unabhängig voneinander für 0, 1, 2, 3 oder 4 stehen;
q für 0, 1 oder 2 steht;
R²⁵ für H, C₁₋₆-Alkyl oder C(O)R²⁷ steht;
R²⁶ für H oder C₁₋₆-Alkyl steht;
R²⁷ für H, C₁₋₆-Alkyl, Het⁴ oder -(CH₂)ᵣ-Aryl steht (wobei die beiden letztgenannten Gruppen gegebenenfalls durch einen oder mehrere Substituenten ausgewählt aus -OH, Cyano, Halogen, Amino, Nitro, C₁₋₆-Alkyl und/oder C₁₋₆-Alkoxy (je nach Fall) substituiert und/oder terminiert sind);
Het⁴ für einen fünf- bis zehngliedrigen heterocyclischen Ring mit einem oder mehreren Heteroatomen ausgewählt aus Sauerstoff, Stickstoff und/oder Schwefel steht, der gegebenenfalls auch einen oder mehrere =O-Substituenten umfaßt;
r für 0, 1, 2, 3 oder 4 steht;
und deren Salze, Solvate und Prodrugs;
mit der Massgabe, daß:
(a) R^{5a}, R^{5b}, R^{5c}, R^{5d}, R^{5e} und R^{5f} nicht alle gleichzeitig für H stehen;
(b) R^{5a} und R^{5b} nicht für C₁₋₃-Alkyl stehen, wenn R^{5c}, R^{5d}, R^{5e} und R^{5f} alle für H stehen; und
(c) wenn D für -OH oder -(CH₂)_{c}N(R¹⁰)R¹¹, wobei c für 0 steht, steht:
(i) A nicht für -N(R²⁵)(CH₂)ₘ-, -O(CH₂)ₘ- oder - (CH₂)ₘC(H)(OR²⁵)(CH₂)ₙ- (wobei n für 0 steht) steht; und/oder
(ii) p nicht für 0 steht, wenn B für - (CH₂)ₚ(N(R²⁶)-, -(CH₂)ₚS(O)_{q}- oder - (CH₂)ₚO- steht.

2. Verbindungen nach Anspruch 1, wobei R¹ für gegebenenfalls substituiertes -(CH₂)ₐ-Phenyl steht, wobei a für 0, 1, 2 oder 3 steht, oder für gegebenenfalls substituiertes, gegebenenfalls ungesättigtes, geradkettiges, verzweigtes oder cyclisches C₁₋₈-Alkyl steht (wobei die letztgenannte Gruppe auch durch ein Sauerstoffatom unterbrochen sein kann).

3. Verbindungen nach Anspruch 1 oder 2, wobei R² für H steht.

4. Verbindungen nach einem der vorhergehenden Ansprüche, wobei R³ für H steht.

5. Verbindungen nach einem der vorhergehenden Ansprüche, wobei R⁴ für H oder C₁₋₃-Alkyl steht.

6. Verbindungen nach einem der vorhergehenden Ansprüche, wobei R^{5a} und R^{5b} entweder beide für H oder beide für Methyl stehen.

7. Verbindungen nach einem der vorhergehenden Ansprüche, wobei R^{5c}, R^{5d}, R^{5e} und R^{5f} unabhängig voneinander für H oder C₁₋₂-Alkyl stehen.

8. Verbindungen nach einem der vorhergehenden Ansprüche, wobei R⁶ für einen oder mehrere Substituenten ausgewählt aus C₁₋₆-Alkyl (wobei die Alkylgruppe gegebenenfalls durch eine N(H)C(O)OR^{20a}-Gruppe (in der R^{20a} für C₁₋₅-Alkyl steht) terminiert ist), Cyano, Nitro, Amino, C(O)N(H)R²¹ und/oder -N(H)S(O)₂R²³ steht.

9. Verbindungen nach einem der vorhergehenden Ansprüche, wobei X für O steht.

10. Verbindungen nach einem der vorhergehenden Ansprüche, wobei A für eine Einfachbindung oder geradkettiges oder verzweigtes C₁₋₄-Alkylen steht (wobei diese Gruppe auch gegebenenfalls durch O unterbrochen ist).

11. Verbindungen nach einem der vorhergehenden Ansprüche, wobei B für eine Einzelbindung, C₁₋₄-Alkylen, -(CH₂)ₚO- oder -(CH₂)ₚN(R²⁶)- steht (wobei in den beiden letztgenannten Fällen p für 1, 2 oder 3 steht).

12. Verbindungen nach einem der vorhergehenden Ansprüche, wobei D für H, OR⁹ (wobei R⁹ für H, C₁₋₃-Alkyl oder gegebenenfalls substituiertes Phenyl steht) oder N(H)R¹⁰ (wobei R¹⁰ für H oder C₁₋₄-Alkyl steht) steht.

13. Pharmazeutische Formulierung, enthaltend eine Verbindung nach einem der Ansprüche 1 bis 12 in einer Mischung mit einem pharmazeutisch unbedenklichen Adjuvans, Verdünnungsmittel oder Träger.

14. Verbindungen nach einem der Ansprüche 1 bis 12 zur Verwendung als Pharmazeutikum.

15. Verbindungen nach einem der Ansprüche 1 bis 12 zur Verwendung bei der Prophylaxe oder der Behandlung einer Arrhythmie.

16. Verwendung einer Verbindung nach einem der Ansprüche 1 bis 12 als Wirkstoff bei der Herstellung eines Medikaments zur Verwendung bei der Prophylaxe oder der Behandlung von Arrhythmie.

17. Verwendung nach Anspruch 16, wobei es sich bei der Arrhytmie um Vorhofarrhythmie oder ventrikuläre Arrhythmie handelt.

18. Verfahren zur Herstellung einer Verbindung der Formel I nach Anspruch 1, bei dem man:
(a) eine Verbindung der Formel II 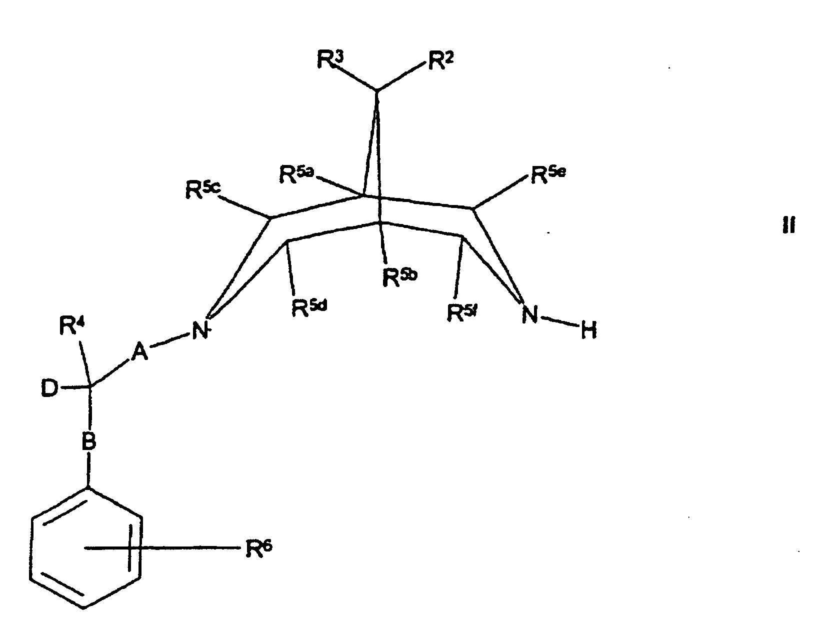 in welcher R², R³, R⁴, R^{5a}, R^{5b}, R^{5c}, R^{5d}, R^{5e}, R^{5f}, R⁶, A, B und D wie in Anspruch 1 definiert sind, mit einer Verbindung der Formel III
R¹XC(O)L¹, III
in welcher L¹ für eine Abgangsgruppe steht und R¹ und X wie in Anspruch 1 definiert sind, umsetzt;
(b) bei Verbindungen der Formel I, in denen A für CH₂ und D für -OH oder -N(H)R¹⁰ steht, wobei R¹⁰ wie in Anspruch 1 definiert ist, eine Verbindung der Formel IV 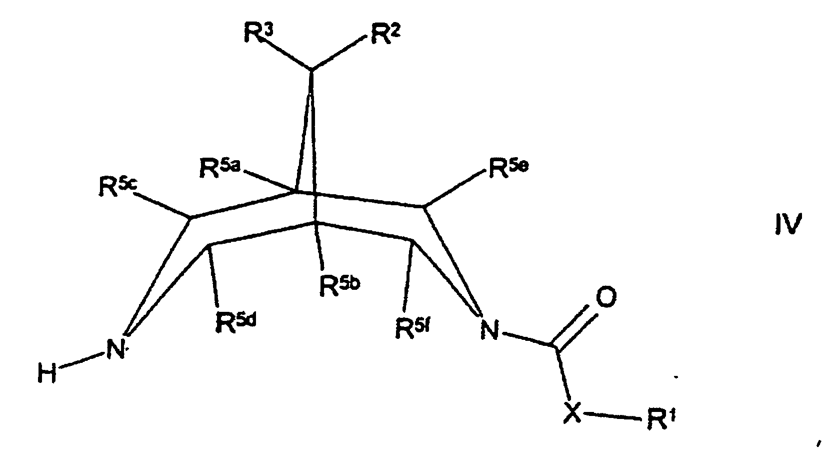 in welcher R¹, R², R³, R^{5a}, R^{5b}, R^{5c}, R^{5d}, R^{5e}, R^{5f} und X wie in Anspruch 1 definiert sind, mit einer Verbindung der Formel V 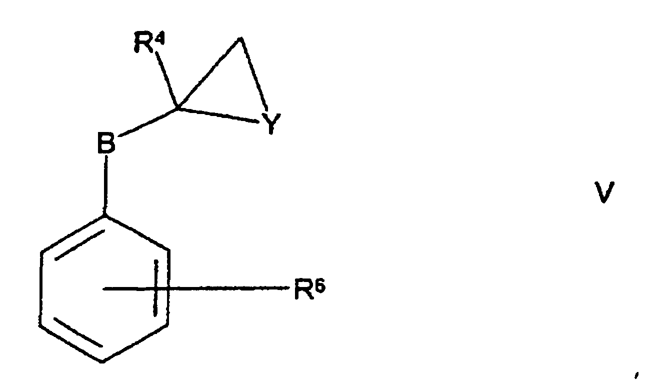 in welcher Y für O oder N(R¹⁰) steht und R⁴, R⁶, R¹⁰ und B wie in Anspruch 1 definiert sind,
umsetzt;
(c) eine wie oben definierte Verbindung der Formel IV mit einer Verbindung der Formel VI 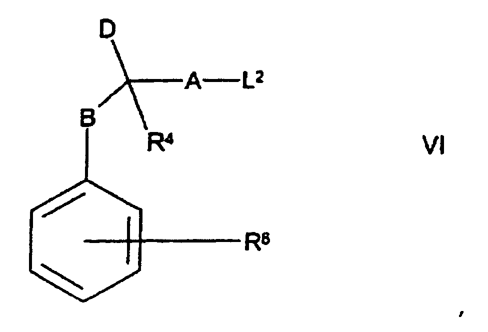 in welcher L² für eine Abgangsgruppe steht und R⁴, R⁶, A, B und D wie in Anspruch 1 definiert sind,
umsetzt;
(d) bei Verbindungen der Formel I, in denen D für H oder OH steht und R⁴ für H steht, eine Verbindung der Formel VII 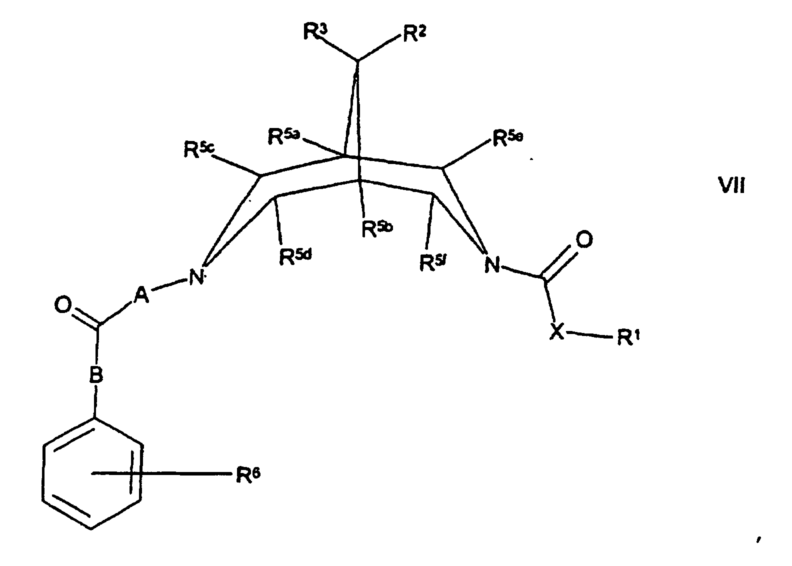 in welcher welcher R¹, R², R³, R^{5a}, R^{5b}, R^{5c}, R^{5d}, R^{5e}, R^{5f}, R⁶, A, B und X wie in Anspruch 1 definiert sind,
reduziert;
(e) bei Verbindungen der Formel I, in denen einer der Reste R² und R³ für H oder OH steht und der andere Rest für H steht, eine entsprechende Verbindung der Formel VIII 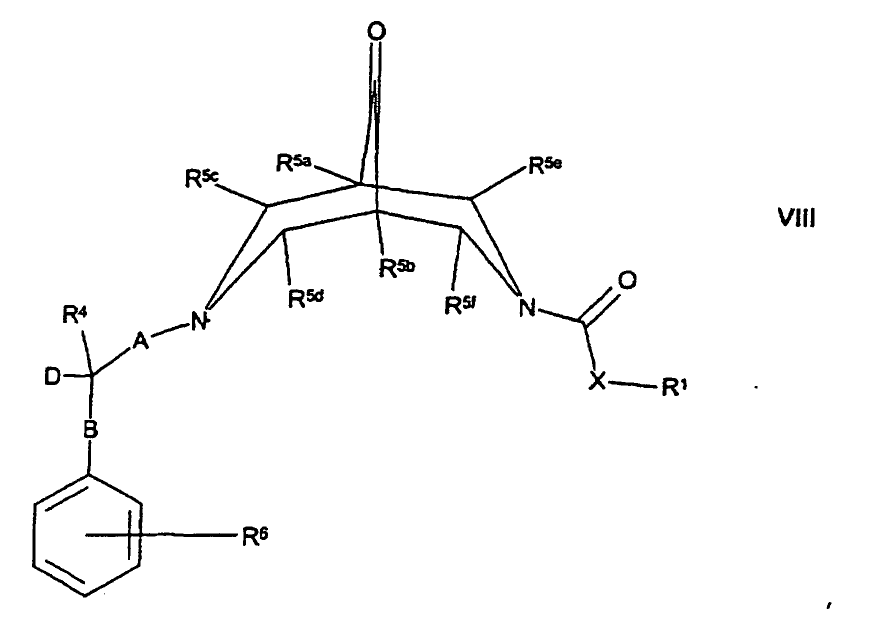 in welcher R¹, R⁴, R^{5a}, R^{5b}, R^{5c}, R^{5d}, R^{5e}, R^{5f}, R⁶, A, B, D und X wie in Anspruch 1 definiert sind,
reduziert;
(f) bei Verbindungen der Formel I, in denen R² und/oder R³ für OC(O)R⁸ stehen/steht und R⁸ wie in Anspruch 1 definiert ist, eine entsprechende Verbindung der Formel I in der
R² und/oder R³ (je nach Fall) für OH stehen/steht und eine Verbindung der Formel VIIIA
R⁸CO₂H, VIIIA
in welcher R⁸ wie in Anspruch 1 definiert ist,
kuppelt;
(g) bei Verbindungen der Formel I, in denen D für - (CH₂)_{c}NH₂ steht, eine entsprechende Verbindung der Formel IX 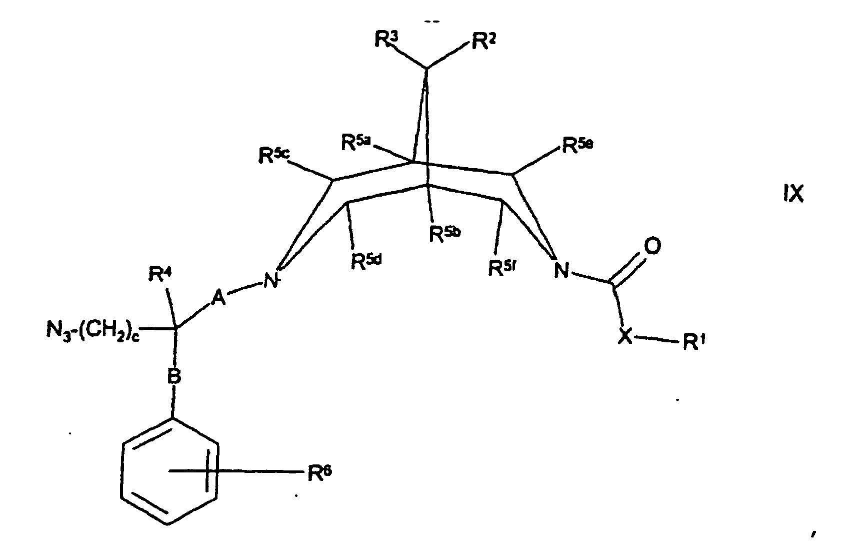 in welcher c, R¹, R², R³, R⁴, R^{5a}, R^{5b}, R^{5c}, R^{5d}, R^{5e}, R^{5f}, R⁶, A, B und X wie in Anspruch 1 definiert sind,
reduziert;
(h) bei Verbindungen der Formel I, in denen D für - N(R¹¹)C(O)NH(R¹⁷) steht, wobei R¹¹ und R¹⁷ wie in Anspruch 1 definiert sind, R¹¹ jedoch nicht für C(O)R²⁰ steht, eine entsprechende Verbindung der Formel I, in der D für -N(R¹¹)H steht, wobei R¹¹ wie in Anspruch 1 definiert ist, jedoch nicht für C(O)R²⁰ steht, wobei R²⁰ wie in Anspruch 1 definiert ist, mit einer Verbindung der Formel X
R¹⁷N=C=O, X
in welcher R¹⁷ wie in Anspruch 1 definiert ist,
umsetzt;
(i) bei Verbindungen der Formel I, in denen D für -N(H)[C(O)]₂NH₂ steht, eine entsprechende Verbindung der Formel I, in der D für -NH₂ steht, mit Oxalsäurediamid umsetzt;
(j) bei Verbindungen der Formel I, in denen D für -N(R¹¹)C(O)R¹⁸ steht, wobei R¹¹ und R¹⁸ wie in Anspruch 1 definiert sind, R¹¹ jedoch nicht für C(O)R²⁰ steht, eine entsprechende Verbindung der Formel I, in der D für -N(R¹¹)H steht, wobei R¹¹ wie in Anspruch 1 definiert ist, jedoch nicht für C(O)R²⁰ steht, mit einer Verbindung der Formel XI
R¹⁸C(O)R^{x}, XI
in welcher R^{x} für eine geeignete Abgangsgruppe steht und R¹⁸ wie in Anspruch 1 definiert ist,
umsetzt;
(k) bei Verbindungen der Formel I, in denen D für -N(H)R¹⁰ steht und R¹⁰ wie in Anspruch 1 definiert ist, jedoch nicht für H oder -C(NH)NH₂ steht, eine entsprechende Verbindung der Formel I, in der D für -NH₂ steht, mit einer Verbindung der Formel XIA
R^{10a}L¹, XIA
in welcher R^{10a} für wie in Anspruch 1 definiertes R¹⁰ steht, jedoch nicht für H oder -C(NH)NH₂, und L¹ wie oben definiert ist,
umsetzt;
(l) bei Verbindungen der Formel I, bei denen es sich um Bispidin-Stickstoff-N-Oxidderivate handelt, den entsprechenden Bispidin-Stickstoff einer entsprechenden Verbindung der Formel I oxidiert;
(m) bei Verbindungen der Formel I, bei denen es sich um quaternäre C₁₋₄-Alkylammoniumsalzderivate handelt, in denen die Alkylgruppe an einen Bispidin-Stickstoff gebunden ist, eine entsprechende Verbindung der Formel I am Bispidin-Stickstoff mit einer Verbindung der Formel XII
R^{a}Hal, XII
in welcher R^{a} für C₁₋₄-Alkyl steht und Hal für Cl, Br oder I steht,
umsetzt;
(n) bei Verbindungen der Formel I, in denen D und R⁴ beide für H stehen, A für C₁₋₆-Alkylen steht, B für -N(R²⁶) (CH₂)ₚ- und R²⁶ und p wie in Anspruch 1 definiert sind, eine Verbindung der Formel XIII 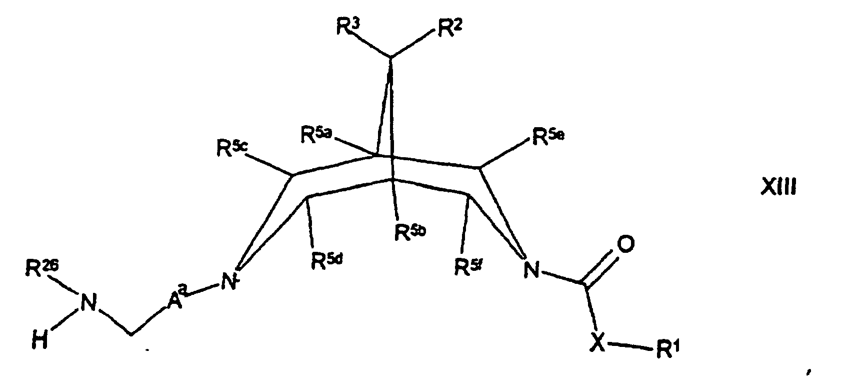 in welcher A^{a} für C₁₋₆-Alkylen steht und R¹, R², R³, R^{5a}, R^{5b}, R^{5c}, R^{5d}, R^{5e}, R^{5f}, R²⁶ und X wie in Anspruch 1 definiert sind, mit einer Verbindung der Formel XIV 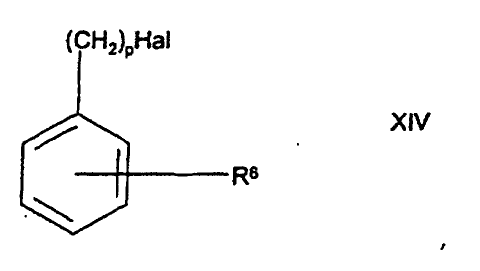 wobei R⁶ und p wie in Anspruch 1 definiert sind und Hal wie oben definiert ist,
umsetzt;
(o) eine wie oben definierte Verbindung der Formel II in Gegenwart von 1,1'-Carbonyldiimidazol mit einer Verbindung der Formel XV
R¹XH, XV
in welcher R¹ und X wie in Anspruch 1 definiert sind,
umsetzt;
(p) bei Verbindungen der Formel I, in denen R⁹ für gegebenenfalls substituiertes C₁₋₆-Alkyl, gegebenenfalls substituiertes -(CH₂)_{d}-Aryl oder gegebenenfalls substituiertes - (CH₂)_{d}-Het² steht, eine entsprechende Verbindung der Formel I, in der D für OH steht, mit einer Verbindung der Formel XVI
R^{9a}OH, XVI
in welcher R^{9a} für gegebenenfalls substituiertes C₁₋₆-Alkyl, gegebenenfalls substituiertes -(CH₂)_{d}-Aryl oder gegebenenfalls substituiertes - (CH₂)_{d}-Het² steht und d und Het² wie in Anspruch 1 definiert sind,
umsetzt;
(q) bei Verbindungen der Formel I, in denen R⁹ für gegebenenfalls substituiertes C₁₋₆-Alkyl, gegebenenfalls substituiertes -(CH₂)_{d}-Aryl oder gegebenenfalls substituiertes - (CH₂)_{d}-Het² steht, eine Verbindung der Formel XVII 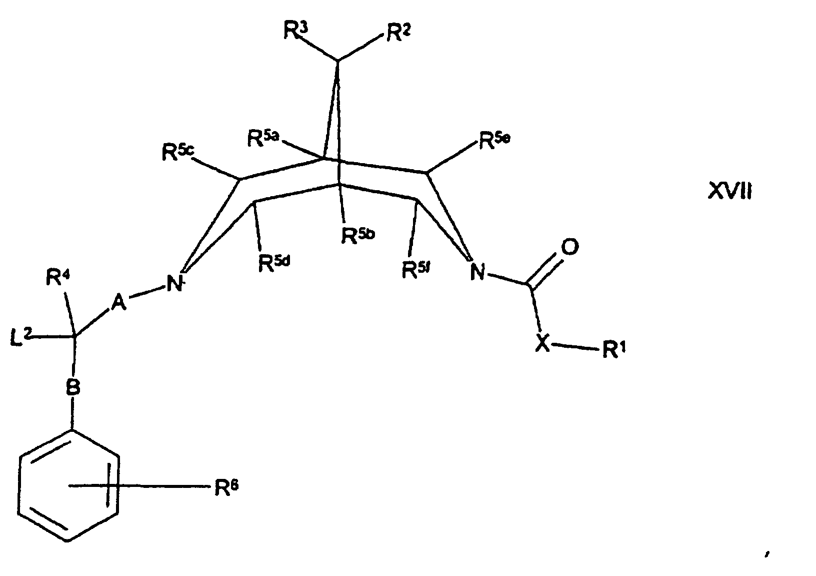 wobei L² wie oben definiert ist und R¹, R², R³, R⁴, R^{5a}, R^{5b}, R^{5c}, R^{5d}, R^{5e}, R^{5f}, R⁶, X, A und B wie in Anspruch 1 definiert sind, mit einer wie oben definierten Verbindung der Formel XVI umsetzt;
(r) bei Verbindungen der Formel I, in denen R⁹ für C(O)R¹² steht und R¹² wie in Anspruch 1 definiert ist, eine entsprechende Verbindung der Formel I, in der D für OH steht, mit einer Verbindung der Formel XVIII
R¹²CO₂H, XVIII
in welcher R¹² wie in Anspruch 1 definiert ist,
umsetzt;
(s) bei Verbindungen der Formel I, in denen einer oder beide der Reste R² und R³ für -N(R^{7a})R^{7b} stehen, wobei einer oder beide der Reste R^{7a} und R^{7b} für C₁₋₆-Alkyl stehen, eine entsprechende Verbindung der Formel I, in der R² und/oder R³ (je nach Fall) für -N(R^{7a})R^{7b} stehen, wobei R^{7a} und/oder R^{7b} (je nach Fall) für H stehen, unter Verwendung einer Verbindung der Formel XVIIIA
R^{7c}L1 XVIIIA
in welcher R^{7c} für C₁₋₆-Alkyl steht und L¹ wie oben definiert ist,
alkyliert;
(t) einen R⁶-Substituenten in einen anderen R⁶-Substituenten umwandelt; oder
(u) ein geschütztes Derivat einer wie in Anspruch 1 definierten Verbindung der Formel I entschützt.

19. Verbindung der Formel II, wie in Anspruch 18 definiert.

20. Verbindungen der Formel IV, wie in Anspruch 18 definiert.

21. Verbindungen der Formel VIII, wie in Anspruch 18 definiert.

22. Verbindungen der Formel XX 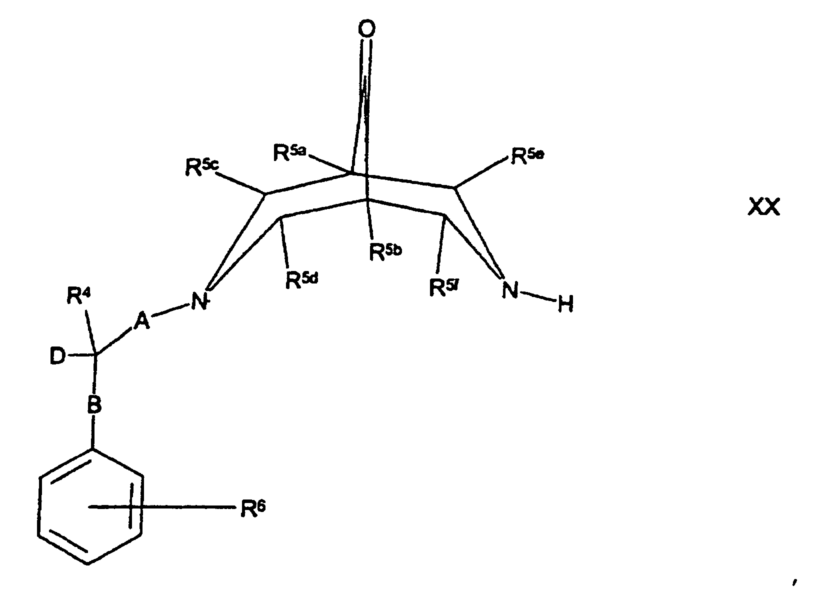 wobei R⁴ , R^{5a}, R^{5b}, R^{5c}, R^{5d}, R^{5e}, R^{5f}, R⁶, A, B und D wie in Anspruch 1 definiert sind.

23. Verbindungen der Formel XXII 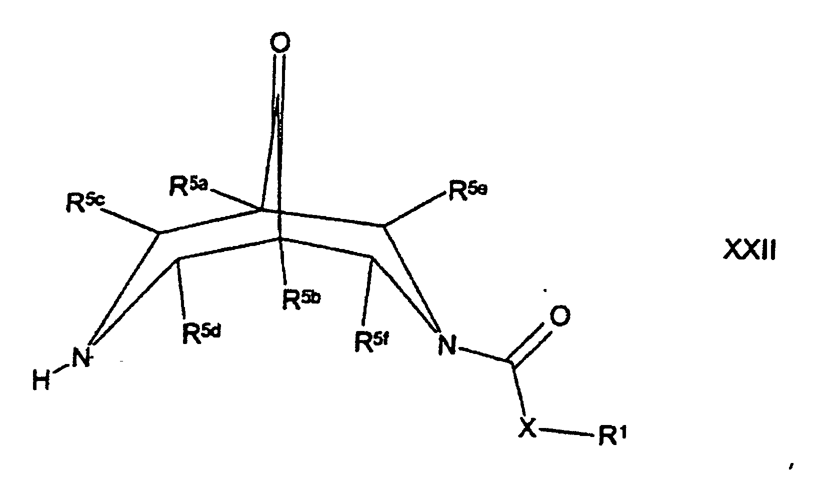 wobei R¹, R^{5a}, R^{5b}, R^{5c}, R^{5d}, R^{5e}, R^{5f} und X wie in Anspruch 1 definiert sind.

24. Verfahren zur Herstellung einer wie in Anspruch 18 definierten Verbindung der Formel VIII, einer wie in Anspruch 22 definierten Verbindung der Formel XX, einer wie in Anspruch 23 definierten Verbindung der Formel XXII oder einer Verbindung der Formel XXXV 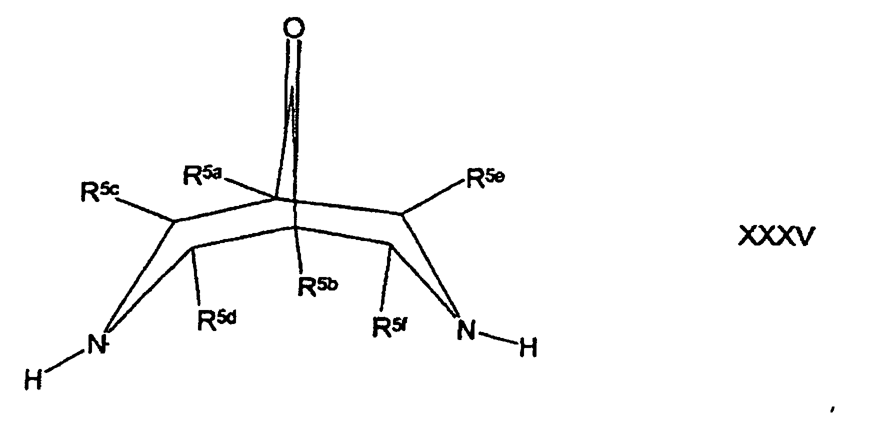 wobei R^{5a} bis R^{5f} wie in Anspruch 1 definiert sind und wobei in allen Fällen R^{5c} und R^{5d} beide für H stehen, bei dem man eine Verbindung der Formel XXXVI 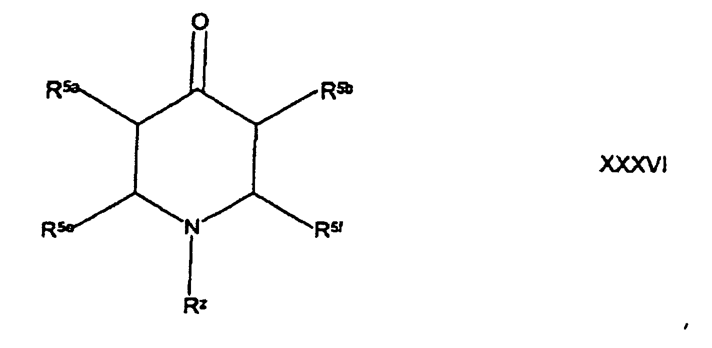 in welcher R^{z} für H oder -C(O)XR¹ steht und R¹, R^{5a}, R^{5b}, R^{5e}, R^{5f} und X wie in Anspruch 1 definiert sind oder ein geschütztes Derivat davon mit (je nach Fall) entweder:
(1) einer Verbindung der Formel XXXVII 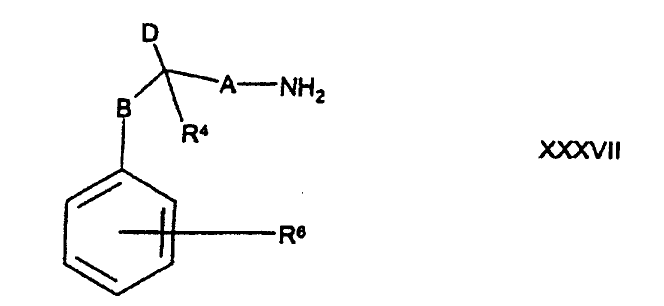 oder einem geschützten Derivat davon, wobei R⁴, R⁶, A, B und D wie in Anspruch 1 definiert sind; oder
(2) NH₃ (oder einem geschützten Derivat davon)
umsetzt, jeweils in Gegenwart von Formaldehyd.

## Revendications

1. Composé de formule I, 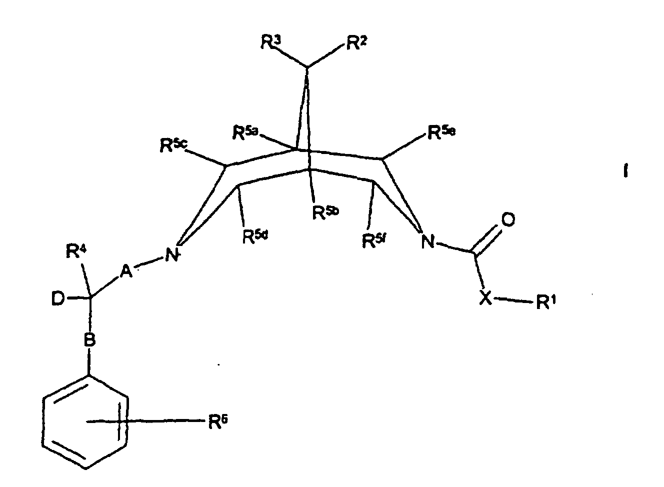 dans laquelle
R¹ représente un alkyle en C₁₋₁₂, un -(CH₂)ₐ-aryle, ou un - (CH₂)ₐ-Het¹ (tous sont éventuellement substitués et/ou terminés (selon le cas) par un ou plusieurs substituants choisis parmi -OH, un halogéno, un cyano, un nitro, un alkyle en C₁₋₄ et/ou un alcoxy en C₁₋₄) ;
a vaut 0, 1, 2, 3 ou 4 ;
Het¹ représente un noyau hétérocyclique à cinq à dix chaînons renfermant un ou plusieurs hétéroatomes choisis parmi un oxygène, un azote et/ou un soufre, et renfermant également éventuellement un ou plusieurs substituants =O ;
X représente O ou S ;
R^{5a}, R^{5b}, R^{5c}, R^{5d}, R^{5e} et R^{5f} représentent indépendamment H ou un alkyle en C₁₋₃ ;
R² et R³ représentent indépendamment H, un alkyle en C₁₋₄ (éventuellement substitué et/ou terminé par un ou plusieurs groupes nitro ou cyano), OR⁷, N(R^{7a})R^{7b}, OC(O)R⁸, ou forment conjointement -O-(CH₂)₂-O-, -(CH₂)₃-, -(CH₂)₄- ou -(CH₂)₅- ;
R⁷ et R⁸ représentent indépendamment H, un alkyle en C₁₋₆ ou un -(CH₂)_{b}-aryle (ces deux derniers groupes sont éventuellement substitués et/ou terminés par un ou plusieurs substituants choisis parmi -OH, un halogéno, un cyano, un nitro, un alkyle en C₁₋₄ et/ou un alcoxy en C₁₋₄) ;
R^{7a} et R^{7b} représentent indépendamment H ou un alkyle en C₁₋₆ ;
b vaut 0, 1, 2, 3 ou 4 ;
R⁴ représente H ou un alkyle en C₁₋₆ ;
D représente H, un alkyle en C₁₋₄, -OR⁹, ou -(CH₂)_{c}N(R¹⁰)(R¹¹) ;
R⁹ représente H, un alkyle en C₁₋₆, -C(O)R¹², -(CH₂)_{d}-aryle ou -(CH₂)_{d}-Het² (ces trois derniers groupes sont éventuellement substitués par un ou plusieurs substituants choisis parmi -OH, un halogéno, un cyano, un nitro, un alkyle en C₁₋₄, un alcoxy en C₁₋₄, C(O)R¹³, C(O)OR¹⁴ et/ou -N(H)S(O)ₑR¹⁵) ;
R¹⁰ représente H, un alkyle en C₁₋₆, un -(CH₂)_{f}-aryle, - C(NH)NH₂, -S(O)₂R^{15a}, -[C(O)]_{g}N(R¹⁶)(R¹⁷), -C(O)R¹⁸ ou -C(O)OR¹⁹ ;
e vaut 0, 1, ou 2 ;
g vaut 1 ou 2 ;
R¹¹ représente H, un alkyle en C₁₋₆, -C(O)R²⁰ ou un -(CH₂)ₕ-aryle (ce dernier groupe est éventuellement substitué et/ou terminé (selon le cas) par un ou plusieurs substituants choisis parmi -OH, un cyano, un halogéno, un amino, un nitro, un alkyle en C₁₋₆ et/ou un alcoxy en C₁₋₆) ;
R¹², R¹³, R¹⁴, R¹⁶, R¹⁷, R¹⁸, R¹⁹ et R²⁰ représentent indépendamment H, un alkyle en C₁₋₆, Het³ ou un -(CH₂)ⱼ-aryle (ces trois derniers groupes sont éventuellement substitués et/ou terminés (selon le cas) par un ou plusieurs substituants choisis parmi -OH, un cyano, un halogéno, un amino, un nitro, un alkyle en C₁₋₆ et/ou un alcoxy en C₁₋₆) ;
R¹⁵ et R^{15a} représentent indépendamment un alkyle en C₁₋₆, un aryle ou un -(CH₂)ₖ-aryle (tous sont éventuellement substitués et/ou terminés (selon le cas) par un ou plusieurs substituants choisis parmi un halogéno, un nitro, un alkyle en C₁₋₆ et/ou un alcoxy en C₁₋₆) ;
c, d, f, h, j et k valent indépendamment 0, 1, 2, 3 ou 4 ;
Het² et Het³ représentent indépendamment des noyaux hétérocycliques à cinq à dix chaînons renfermant un ou plusieurs hétéroatomes choisis parmi un oxygène, un azote et/ou un soufre, et renfermant également éventuellement un ou plusieurs substituants =O ;
R⁶ représente H ou un ou plusieurs substituants choisis parmi -OH, un cyano, un halogéno, un amino, un nitro, un alkyle en C₁₋₆ (éventuellement terminé par N(H)C(O)OR^{20a}), un alcoxy en C₁₋₆, -C(O)N(H)R²¹, -NHC(O)N(H)R²², -N(H)S(O)₂R²³ et/ou -OS(O)₂R²⁴ ;
R²¹ et R²² représentent indépendamment H ou un alkyle en C₁₋₆ ;
R^{20a}, R²³ et R²⁴ représentent indépendamment un alkyle en C₁₋₆ ;
A représente une liaison simple, un alkylène en C₁₋₆, -N(R²⁵)(CH₂)ₘ-, -O(CH₂)ₘ- ou -(CH₂)ₘC(H)(OR²⁵)(CH₂)ₙ- (dans ces trois derniers groupes, le groupe -(CH₂)ₘ- est fixé à l'atome d'azote de bispidine et ces quatre derniers groupes sont éventuellement substitués par un ou plusieurs groupes -OH) ;
B représente une liaison simple, un alkylène en C₁₋₄, - (CH₂)ₚN(R²⁶)-, -(CH₂)ₚS(O)_{q}-, -(CH₂)ₚO- (dans ces trois derniers groupes, le groupe -(CH₂)ₚ- est relié à l'atome de carbone portant D et R⁴), -C(O)N(R²⁶)- (dans ce dernier groupe, le groupe -C(O)- est relié à l'atome de carbone portant D et R⁴), -N(R²⁶)C(O)O(CH₂)ₚ- ou -N(R²⁶)(CH₂)ₚ (dans ces deux derniers groupes, le groupe N(R²⁶) est relié à l'atome de carbone portant D et R⁴) ;
m, n et p valent indépendamment 0, 1, 2, 3 ou 4 ;
q vaut 0, 1 ou 2 ;
R²⁵ représente H, un alkyle en C₁₋₆ ou C(O)R²⁷ ;
R²⁶ représente H ou un alkyle en C₁₋₆ ;
R²⁷ représente H, un alkyle en C₁₋₆, Het⁴ ou un -(CH₂)ᵣ-aryle (ces deux derniers groupes sont éventuellement substitués et/ou terminés (selon le cas) par un ou plusieurs substituants choisis parmi -OH, un cyano, un halogéno, un amino, un nitro, un alkyle en C₁₋₆ et/ou un alcoxy en C₁₋₆) ;
Het⁴ représente un noyau hétérocyclique à cinq à dix chaînons renfermant un ou plusieurs hétéroatomes choisis parmi un oxygène, un azote et/ou un soufre, et renfermant également éventuellement un ou plusieurs substituants =O ;
r vaut 0, 1, 2, 3 ou 4 ;
ou un sel, un solvate ou un promédicament de celui-ci ;
à condition que :
(a) R^{5a}, R^{5b}, R^{5c}, R^{5d}, R^{5e} et R^{5f} ne représentent pas tous simultanément H ;
(b) R^{5a} et R^{5b} ne représentent pas un alkyle en C₁₋₃ lorsque R^{5c}, R^{5d}, R^{5e} et R^{5f} représentent tous H ; et
(c) lorsque D représente -OH ou -(CH₂)_{C}N(R¹⁰)R¹¹ dans lequel c vaut 0, alors :
(i) A ne représente pas -N(R²⁵)(CH₂)ₘ-, -O(CH₂)ₘ- ou -(CH₂)ₘC(H)(OR²⁵)(CH₂)ₙ- (dans lequel n vaut 0) ; et/ou
(ii) p ne vaut pas 0 lorsque B représente -(CH₂)ₚN(R²⁶)-, -(CH₂)ₚS(O)_{q}- ou -(CH₂)ₚO-.

2. Composé selon la revendication 1, dans lequel R¹ représente un -(CH₂)ₐ-phényle éventuellement substitué, dans lequel a vaut 0, 1, 2 ou 3, ou un alkyle en C₁₋₈ linéaire, ramifié ou cyclique, éventuellement insaturé et éventuellement substitué (ce dernier groupe peut également être interrompu par un atome d'oxygène).

3. Composé selon la revendication 1 ou la revendication 2, dans lequel R² représente H.

4. Composé selon l'une quelconque des revendications précédentes, dans lequel R³ représente H.

5. Composé selon l'une quelconque des revendications précédentes, dans lequel R⁴ représente H ou un alkyle en C₁₋₃.

6. Composé selon l'une quelconque des revendications précédentes, dans lequel R^{5a} et R^{5b} soit représentent tous les deux H, soit représentent tous les deux un méthyle.

7. Composé selon l'une quelconque des revendications précédentes, dans lequel R^{5c}, R^{5d}, R^{5e} et R^{5f} représentent indépendamment H ou un alkyle en C₁₋₂.

8. Composé selon l'une quelconque des revendications précédentes, dans lequel R⁶ représente un ou plusieurs substituants choisis parmi un alkyle en C₁₋₆ (ce groupe alkyle est éventuellement terminé par un groupe N(H)C(O)OR^{20a} (dans lequel R^{20a} représente un alkyle en C₁₋₅)), un cyano, un nitro, un amino, C(O)N(H)R²¹ et/ou -N(H)S(O)₂R²³.

9. Composé selon l'une quelconque des revendications précédentes, dans lequel X représente O.

10. Composé selon l'une quelconque des revendications précédentes, dans lequel A représente une liaison simple ou un alkylène en C₁₋₄ linéaire ou ramifié (ce groupe est également éventuellement interrompu par O).

11. Composé selon l'une quelconque des revendications précédentes, dans lequel B représente une liaison simple, un alkylène en C₁₋₄, -(CH₂)ₚO- ou -(CH₂)ₚN(R²⁶)-(dans ces deux derniers cas, p vaut 1, 2 ou 3).

12. Composé selon l'une quelconque des revendications précédentes, dans lequel D représente H, OR⁹ (dans lequel R⁹ représente H, un alkyle en C₁₋₃ ou un phényle éventuellement substitué) ou N(H)R¹⁰ (dans lequel R¹⁰ représente H ou un alkyle en C₁₋₄).

13. Formulation pharmaceutique comprenant un composé tel que défini dans l'une quelconque des revendications 1 à 12 en mélange avec un adjuvant, un diluant ou un support pharmaceutiquement acceptable.

14. Composé tel que défini dans l'une quelconque des revendications 1 à 12, destiné à être utilisé en tant qu'agent pharmaceutique.

15. Composé tel que défini dans l'une quelconque des revendications 1 à 12, destiné à être utilisé pour la prophylaxie ou le traitement d'une arythmie.

16. Utilisation d'un composé tel que défini dans l'une quelconque des revendications 1 à 12, en tant qu'ingrédient actif pour la fabrication d'un médicament destiné à être utilisé pour la prophylaxie ou le traitement d'une arythmie.

17. Utilisation selon la revendication 16, dans laquelle l'arythmie est une arythmie atriale ou ventriculaire.

18. Procédé de préparation d'un composé de formule I tel que défini dans la revendication 1, comprenant :
(a) la réaction d'un composé de formule II, 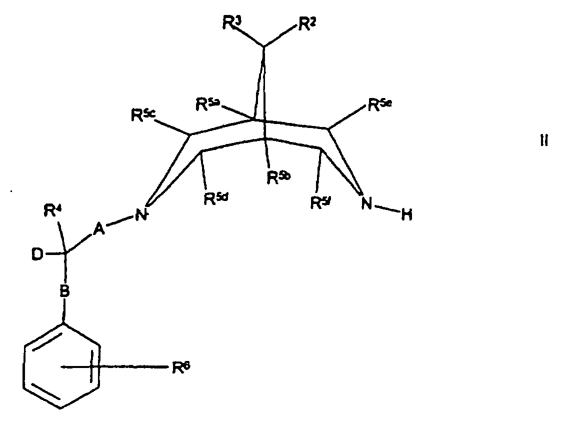 dans laquelle R², R³, R⁴, R^{5a}, R^{5b}, R^{5c}, R^{5d}, R^{5e}, R^{5f}, R⁶, A, B et D sont tels que définis dans la revendication 1, avec un composé de formule III,
R¹XC(O)L¹ III
dans laquelle L¹ représente un groupe partant et R¹ et X sont tels que définis dans la revendication 1 ;
(b) pour des composés de formule I dans laquelle A représente CH₂ et D représente -OH ou -N(H)R¹⁰, dans lequel R¹⁰ est tel que défini dans la revendication 1, la réaction d'un composé de formule IV, 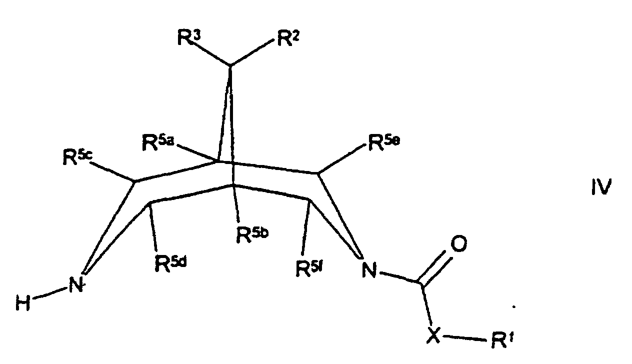 dans laquelle R¹, R², R³, R^{5a}, R^{5b}, R^{5c}, R^{5d}, R^{5e}, R^{5f} et X sont tels que définis dans la revendication 1, avec un composé de formule V, 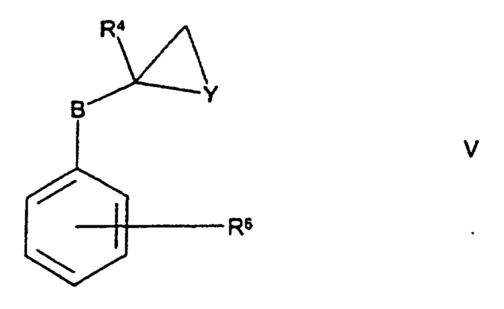 dans laquelle Y représente O ou N(R¹⁰) et R⁴, R⁶, R¹⁰ et B sont tels que définis dans la revendication 1 ;
(c) la réaction d'un composé de formule IV, tel que défini ci-dessus, avec un composé de formule VI, 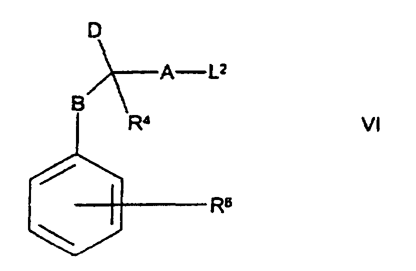 dans laquelle L² représente un groupe partant et R⁴, R⁶, A, B et D sont tels que définis dans la revendication 1 ;
(d) pour des composés de formule I dans laquelle D représente H ou OH et R⁴ représente H, la réduction d'un composé de formule VII, 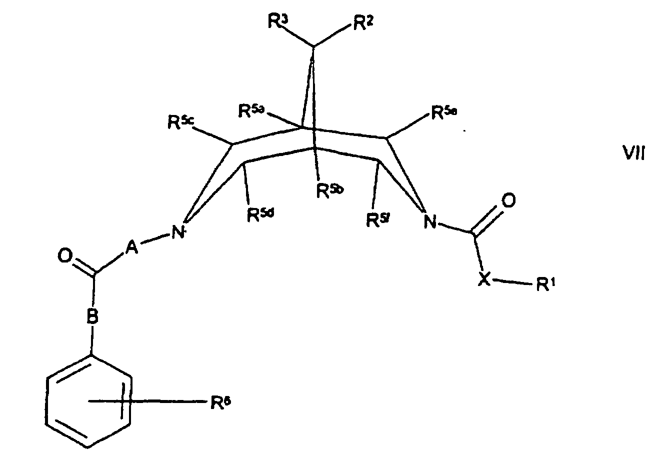 dans laquelle R¹, R², R³, R^{5a}, R^{5b}, R^{5c}, R^{5d}, R^{5e}, R^{5f}, R⁶, A, B, et X sont tels que définis dans la revendication 1 ;
(e) pour des composés de formule I dans laquelle l'un parmi R² et R³ représente H ou OH et l'autre représente H, la réduction d'un composé correspondant de formule VIII, 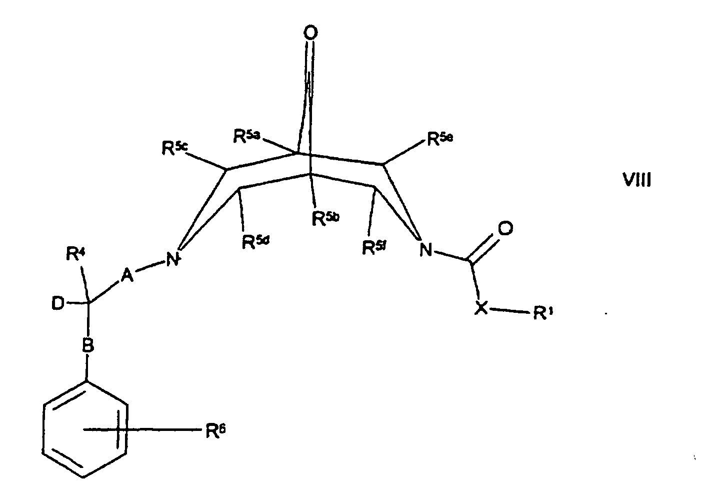 dans laquelle R¹, R⁴, R^{5a}, R^{5b}, R^{5c}, R^{5d}, R^{5e}, R^{5f}, R⁶, A, B, D et X sont tels que définis dans la revendication 1 ;
(f) pour des composés de formule I dans laquelle R² et/ou R³ représentent OC(O)R⁸ et R⁸ est tel que défini dans la revendication 1, le couplage d'un composé correspondant de formule I dans laquelle R² et/ou R³ (selon le cas) représentent OH, et d'un composé de formule VIIIA,
R⁸CO₂H VIIIA
dans laquelle R⁸ est tel que défini dans la revendication 1 ;
(g) pour des composés de formule I dans laquelle D représente -(CH₂)_{C}NH₂, la réduction d'un composé correspondant de formule IX, 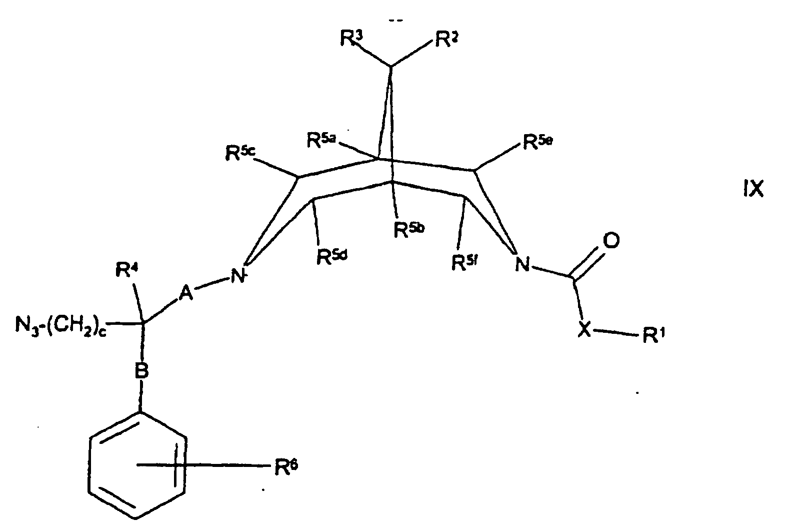 dans laquelle c, R¹, R², R³, R⁴, R^{5a}, R^{5b}, R^{5c}, R^{5d}, R^{5e}, R^{5f}, R⁶, A, B et X sont tels que définis dans la revendication 1 ;
(h) pour des composés de formule I dans laquelle D représente -N(R¹¹)C(O)NH(R¹⁷), dans lequel R¹¹ et R¹⁷ sont tels que définis dans la revendication 1, excepté que R¹¹ ne représente pas C(O)R²⁰, la réaction d'un composé correspondant de formule I dans laquelle D représente -N(R¹¹)H, dans lequel R¹¹ est tel que défini dans la revendication 1, excepté qu'il ne représente pas C(O)R²⁰ dans lequel R²⁰ est tel que défini dans la revendication 1, avec un composé de formule X,
R¹⁷N=C=O X
dans laquelle R¹⁷ est tel que défini dans la revendication 1 ;
(i) pour des composés de formule I dans laquelle D représente -N(H)[C(O)]₂NH₂, la réaction d'un composé correspondant de formule I dans laquelle D représente -NH₂, avec le diamide d'acide oxalique ;
(j) pour des composés de formule I dans laquelle D représente -N(R¹¹)C(O)R¹⁸, dans lequel R¹¹ et R¹⁸ sont tels que définis dans la revendication 1, excepté que R¹¹ ne représente pas C(O)R²⁰, la réaction d'un composé correspondant de formule I dans laquelle D représente - N(R¹¹)H, dans lequel R¹¹ est tel que défini dans la revendication 1, excepté qu'il ne représente pas C(O)R²⁰, avec un composé de formule XI,
R¹⁸C(O)R^{x} XI
dans laquelle R^{x} représente un groupe partant approprié et R¹⁸ est tel que défini dans la revendication 1 ;
(k) pour des composés de formule I dans laquelle D représente -N(H)R¹⁰ et R¹⁰ est tel que défini dans la revendication 1, excepté qu'il ne représente pas H ou -C(NH)NH₂, la réaction d'un composé correspondant de formule I dans laquelle D représente -NH₂, avec un composé de formule XIA,
R^{10a}L¹ XIA
dans laquelle R^{10a} représente R¹⁰ tel que défini dans la revendication 1, excepté qu'il ne représente pas H ou -C(NH)NH₂ et L¹ est tel que défini ci-dessus ;
(l) pour des composés de formule I qui sont des dérivés N-oxyde sur l'azote de bispidine, l'oxydation de l'azote de bispidine correspondant d'un composé correspondant de formule I ;
(m) pour des composés de formule I qui sont des dérivés sels d'ammonium quaternaires d'alkyle en C₁₋₄, dans lesquels le groupe alkyle est fixé à un azote de bispidine, la réaction, au niveau de l'azote de bispidine, d'un composé correspondant de formule I avec un composé de formule XII,
R^{a}Hal XII
dans laquelle R^{a} représente un alkyle en C₁₋₄ et Hal représente Cl, Br ou I ;
(n) pour des composés de formule I dans laquelle D et R⁴ représentent tous les deux H, A représente un alkylène en C₁₋₆, B représente -N(R²⁶)(CH₂)ₚ- et R²⁶ et p sont tels que définis dans la revendication 1, la réaction d'un composé de formule XIII, 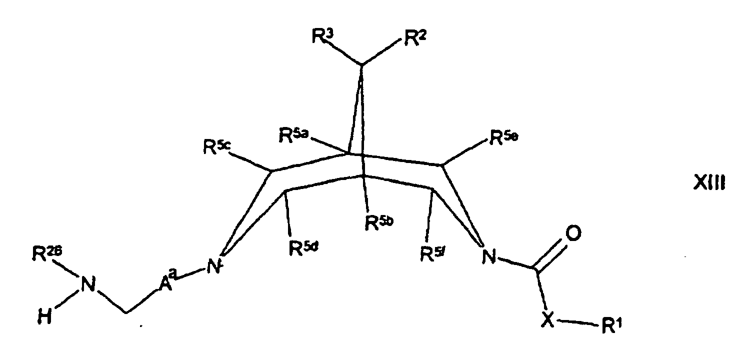 dans laquelle A^{a} représente un alkylène en C₁₋₆ et R¹, R², R³, R^{5a}, R^{5b}, R^{5c}, R^{5d}, R^{5e}, R^{5f}, R²⁶ et X sont tels que définis dans la revendication 1, avec un composé de formule XIV, 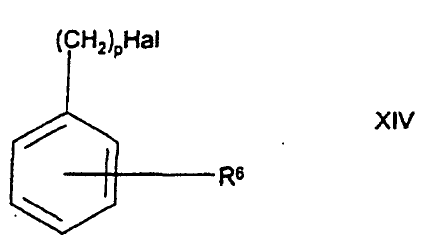 dans laquelle R⁶ et p sont tels que définis dans la revendication 1 et Hal est tel que défini ci-dessus ;
(o) la réaction d'un composé de formule II, tel que défini ci-dessus, avec un composé de formule XV,
R¹XH XV
dans laquelle R¹ et X sont tels que définis dans la revendication 1, en présence d'1,1'-carbonyldiimidazole ;
(p) pour des composés de formule I dans laquelle R⁹ représente un alkyle en C₁₋₆ éventuellement substitué, un -(CH₂)_{d}-aryle éventuellement substitué ou un -(CH₂)_{d}-Het² éventuellement substitué, la réaction d'un composé correspondant de formule I, dans laquelle D représente OH, avec un composé de formule XVI,
R^{9a}OH XVI
dans laquelle R^{9a} représente un alkyle en C₁₋₆ éventuellement substitué, un -(CH₂)_{d}-aryle éventuellement substitué ou un -(CH₂)_{d}-Het² éventuellement substitué, et d et Het² sont tels que définis dans la revendication 1 ;
(q) pour des composés de formule I dans laquelle R⁹ représente un alkyle en C₁₋₆ éventuellement substitué, un -(CH₂)_{d}-aryle éventuellement substitué ou un -(CH₂)_{d}-Het² éventuellement substitué, la réaction d'un composé de formule XVII, 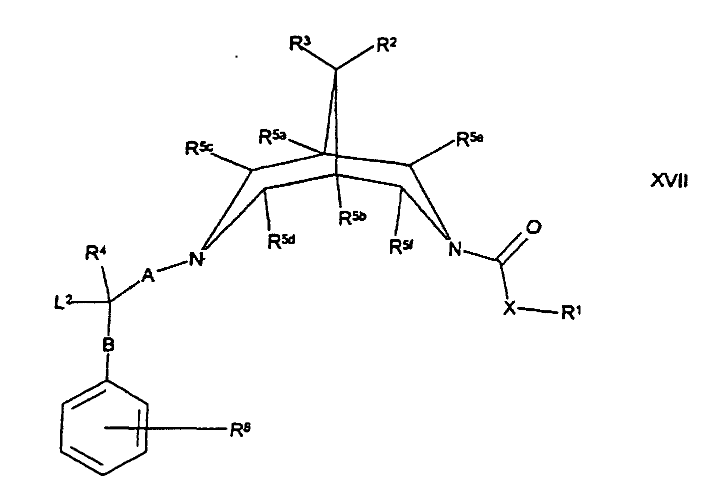 dans laquelle L² est tel que défini ci-dessus et R¹, R², R³, R⁴, R^{5a}, R^{5b}, R^{5c}, R^{5d}, R^{5e}, R^{5f}, R⁶, X, A et B sont tels que définis dans la revendication 1, avec un composé de formule XVI tel que défini ci-dessus ;
(r) pour des composés de formule I dans laquelle R⁹ représente C(O)R¹² et R¹² est tel que défini dans la revendication 1, la réaction d'un composé correspondant de formule I dans laquelle D représente OH, avec un composé de formule XVIII,
R¹²CO₂H XVIII
dans laquelle R¹² est tel que défini dans la revendication 1 ;
(s) pour des composés de formule I dans laquelle l'un ou les deux parmi R² et R³ représentent -N(R^{7a})R^{7b} dans lequel l'un ou les deux parmi R^{7a} et R^{7b} représentent un alkyle en C₁₋₆, l'alkylation d'un composé correspondant de formule I dans laquelle R² et/ou R³ représentent -N(R^{7a})R^{7b} (selon le cas) dans lequel R^{7a} et/ou R^{7b} (selon le cas) représentent H, en utilisant un composé de formule XVIIIA,
R^{7c}L¹ XVIIIA
dans laquelle R^{7c} représente un alkyle en C₁₋₆ et L¹ est tel que défini ci-dessus ;
(t) la transformation d'un substituant R⁶ en un autre ; ou
(u) la déprotection d'un dérivé protégé d'un composé de formule I tel que défini dans la revendication 1.

19. Composé de formule II tel que défini dans la revendication 18.

20. Composé de formule IV tel que défini dans la revendication 18.

21. Composé de formule VIII tel que défini dans la revendication 18.

22. Composé de formule XX, 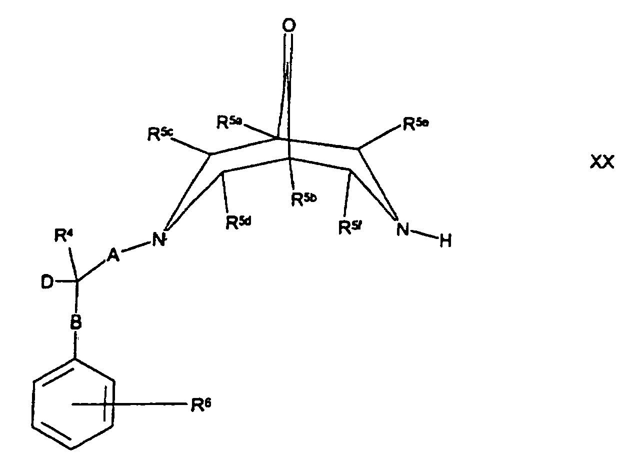 dans laquelle R⁴, R^{5a}, R^{5b}, R^{5c}, R^{5d}, R^{5e}, R^{5f}, R⁶, A, B et D sont tels que définis dans la revendication 1.

23. Composé de formule XXII, 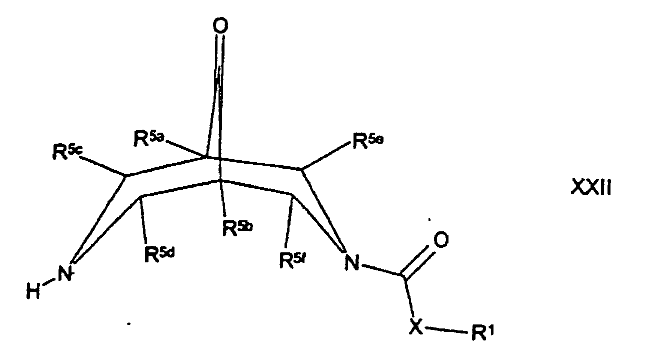 dans laquelle R¹, R^{5a}, R^{5b}, R^{5c}, R^{5d}, R^{5e}, R^{5f} et X sont tels que définis dans la revendication 1.

24. Procédé de préparation d'un composé de formule VIII, tel que défini dans la revendication 18, d'un composé de formule XX, tel que défini dans la revendication 22, d'un composé de formule XXII, tel que défini dans la revendication 23, ou d'un composé de formule XXXV, 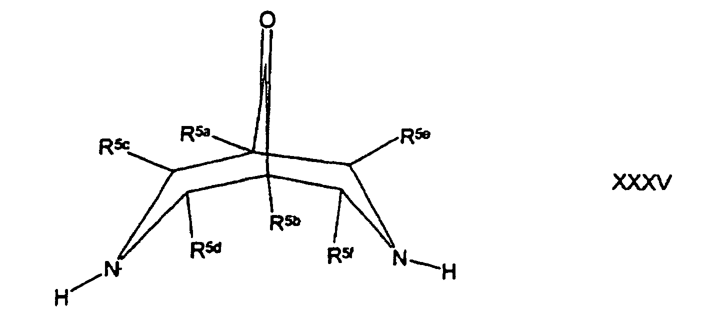 dans laquelle R^{5a} à R^{5f} sont tels que définis dans la revendication 1, et dans lesquels, dans tous les cas, R^{5c} et R^{5d} représentent tous les deux H, lequel procédé comprend la réaction d'un composé de formule XXXVI, 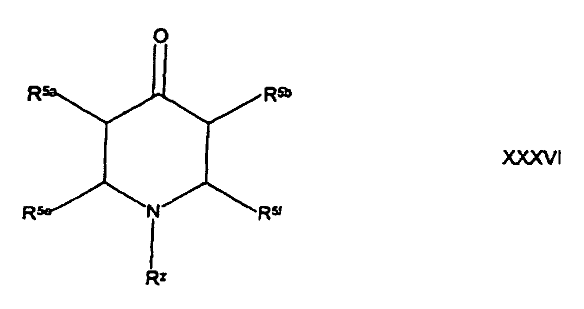 dans laquelle R^{z} représente H ou -C(O)XR¹ et R¹, R^{5a}, R^{5b}, R^{5e}, R^{5f} et X sont tels que définis dans la revendication 1, ou d'un dérivé protégé de celui-ci, avec (selon le cas) soit :
(1) un composé de formule XXXVII, 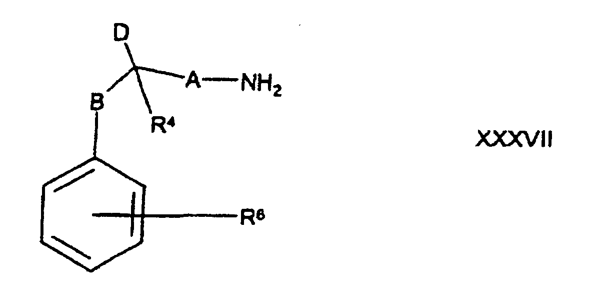 ou un dérivé protégé de celui-ci, dans laquelle R⁴, R⁶, A, B et D sont tels que définis dans la revendication 1 ; ou
(2) NH₃ (ou un dérivé protégé de celui-ci),
dans tous les cas en présence d'un formaldéhyde.
